# EUROPÄISCHE PATENTSCHRIFT

(11) **EP 3 377 030 B1**
(45) Veröffentlichungstag und Bekanntmachung des Hinweises auf die Patenterteilung: **06.05.2020**
(21) Anmeldenummer: 16795380.1
(22) Anmeldetag: 16.11.2016
(51) Int. Cl.: A61K 8/362, A61K 8/44, A61K 8/49, A61Q 5/06, A61Q 5/08, A61Q 5/10, A61K 8/22

(54) **HAAR SCHONENDE MITTEL UND VERFAHREN ZUR OXIDATIVEN HAARFÄRBUNG ODER BLONDIERUNG**
HAIR-CARE AGENT AND METHOD FOR OXIDATIVE HAIR DYING OR BLEACHING
PRODUITS ET PROCÉDÉS DE COLORATION OU DE DÉCOLORATION D'OXYDATION MÉNAGEANT LES CHEVEUX

(30) Priorität: 20.11.2015 DE 102015222946; 31.05.2016 DE 102016209468; 03.06.2016 US 201662345085 P
(43) Veröffentlichungstag der Anmeldung: 26.09.2018
(73) Patentinhaber: Henkel AG & Co. KGaA, 40589 Düsseldorf (DE)
(72) Erfinder: MANNECK, Hartmut, 23858 Barnitz (DE); HIPPE, Thomas, 25482 Appen (DE); KLEEN-FEHRES, Astrid, 20457 Hamburg (DE)
(86) Internationale Anmeldenummer: PCT/EP2016/077846
(87) Internationale Veröffentlichungsnummer: WO 2017/085117

(56) Entgegenhaltungen:
- WO-A1-2005/115314
- WO-A1-2017/102582
- DE-A1- 10 051 774
- US-A1- 2011 247 644

## Beschreibung

Die vorliegende Erfindung betrifft ein Haar schonendes Mittel zur oxidativen Haarfärbung oder Blondierung sowie ein schonendes Verfahren zur oxidativen Haarfärbung oder Blondierung, bei dem keratinische Fasern vor oxidativen Einflüssen geschützt bzw. oxidative Haarschäden repariert werden.

Beim oxidativen Färben oder Blondieren von Haaren tritt das Problem auf, dass es aufgrund der aggressiven Agentien zu Schäden an der keratinischen Faser kommen kann. Insbesondere die natürliche Hydrophobizität der keratinischen Faser wird reduziert, da die Färbe- bzw. Aufhellmittel das Haar zunächst penetrationsfähig machen müssen, um ihre Wirkung zu entfalten. Die wasserabweisende Wirkung ist aber einerseits ein natürlicher Schutz des Haares, andererseits sind vom Verbraucher erwünschte Parameter wie Glanz, Geschmeidigkeit, Griff und "Fallen" der Haare eng mit ihr verknüpft.

Um die genannten Nachteile zu überwinden, sind so genannte Vorbehandlungsmittel auf dem Markt, die das Haar vor dem aggressiven Einfluss schützen sollen. Diese beschweren das Haar aber oft oder beeinträchtigen den Erfolg der im Anschluss stattfindenden Aufhellung bzw. Färbung des Haares; insbesondere die Waschechtheit der Färbung kann durch das Vorbehandlungsmittel verschlechtert sein. Auch sind zahlreiche Nachbehandlungsmittel bekannt, mit denen versucht wird, die bei der oxidativen Färbebehandlung verursachten Haarschädigungen zu reparieren. Alle diese Verfahren erfordern aber ein mehrstufiges Anwendungsverfahren, eben entweder eine dem Färben vor- oder nachgelagerte Applikation eines weiteren Haarbehandlungsmittels. Dies wird vom Verbraucher häufig als lästig wahrgenommen, da bereits die oxidative Färbebehandlung selbst mit mehreren Arbeitsschritten und einer Einwirkzeit von bis zu 60 Minuten sehr aufwändig ist.

Der vorliegenden Erfindung lag die Aufgabe zugrunde, ein Mittel und ein Verfahren zur oxidativen Haarfärbung mit einer Haar schützenden Behandlung bereitzustellen, das die genannten Nachteile überwindet, ohne das Farbergebnis der oxidativen Färbebehandlung negativ zu beeinflussen. Dabei sollten insbesondere ein Färbemittel und ein Verfahren bereitgestellt werden, bei dem das Haar nicht beschwert wird und eine möglichst geringe Haarschädigung auftritt. Weiterhin sollte der erzielte Haarschutz möglichst wenig zeitaufwändig sein und möglichst zusammen mit dem Färbeschritt selbst erfolgen.

Der Einsatz von Dicarbonsäuren wie Bernsteinsäure in der Haarpflege ist Stand der Technik. Diese werden in Shampoos und insbesondere in Conditionern breit eingesetzt, um dort Pflegeeffekte zu entfalten. So offenbart die Patentanmeldung WO 2005/115314A1 ein Verfahren zur Restrukturierung keratinischer Fasern, bei dem die Keratinfasern mit Cystin und mit mindestens einer Dicarbonsäure mit 2 bis 10 Kohlenstoffatomen in Kontakt gebracht werden, wobei bevorzugte Dicarbonsäuren ausgewählt sind aus Oxalsäure, Malonsäure, Bernsteinsäure, Glutarsäure, Adipinsäure, Pimelinsäure, Azelainsäure, Maleinsäure, Fumarsäure und Sorbinsäure und Bernsteinsäure besonders bevorzugt ist. Die Patentanmeldung DE 10051774 A1 beschreibt die Verwendung kurzkettiger Carbonsäuren mit einem Molekulargewicht unter 750 g/mol in kosmetischen Mitteln als Wirkstoff zur Restrukturierung keratinischer Fasern. Die Patentanmeldung EP1174112A offenbart Haarbehandlungsmittel, die neben einer organischen Säure als weitere zwingende Bestandteile ein organisches Lösungsmittel, ein kationisches Tensid und einen höheren Alkohol enthalten und zur Reparatur von Poren in Haaren dienen.

Es wurde nun gefunden, dass oxidative Färbe- und Blondiermittel, die neben typischen Bestandteilen, wie Wasser, Ammoniumhydroxid und/oder Monoethanolamin als Alkalisierungsmittel und einer Peroxidverbindung, wie Wasserstoffperoxid, mindestens eine gesättigte Dicarbonsäure mit 2 bis 10 Kohlenstoffatomen und mindestens eine Aminosäure der Formel (VI) enthalten, zu deutlich verbessertem Haarschutz bei der oxidativen Haarbehandlung führen, ohne dass die Ergebnisse der oxidativen Färbe- oder Blondierbehandlung beeinträchtigt werden. Es wurde überraschend gefunden, dass durch den Gehalt an mindestens einer gesättigten Dicarbonsäure mit 2 bis 10 Kohlenstoffatomen in Kombination mit mindestens einer Aminosäure der Formel (VI) das Haar während der Färbens und/oder des Aufhellens vor Schädigungen durch den hohen pH des Mittels und durch das Oxidationsmittel geschützt wird. Dies ließ sich unter anderem daran feststellen, dass beim anschließenden Kämmen weniger Haarbruch auftrat und das Haar weniger an Elastizität einbüßte, wie sich durch Zug-Dehn-Messungen nachweisen ließ, als nach der Applikation nicht erfindungsgemäßer Färbe- und Blondiermittel.

Gegenstand der vorliegenden Erfindung ist in einer ersten Ausführungsform ein oxidatives Färbe- oder Blondiermittel für keratinische Fasern, insbesondere für Humanhaar, enthaltend
a) mindestens eine gesättigte Dicarbonsäure mit 2 bis 10 Kohlenstoffatomen und/oder mindestens ein Salz dieser Säure(n),
b) mindestens eine Aminosäure der Formel (VI) 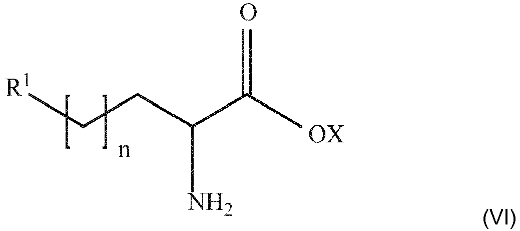 worin
   X für ein Wasserstoffatom oder ein ein- oder zweiwertiges Kation steht;
   n für null, 1, 2 oder 3 steht;
   R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung.
   Ein weiterer Gegenstand der vorliegenden Erfindung ist ein Verfahren zur oxidativen Färbung und/oder Aufhellung keratinischer Fasern, insbesondere von Humanhaar, bei dem ein Färbe- oder Blondiermittel auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird, wobei dieses Färbe- oder Blondiermittel
   a) mindestens eine gesättigte Dicarbonsäure mit 2 bis 10 Kohlenstoffatomen und/oder mindestens ein Salz dieser Säure(n),
   b) mindestens eine Aminosäure der Formel (VI) 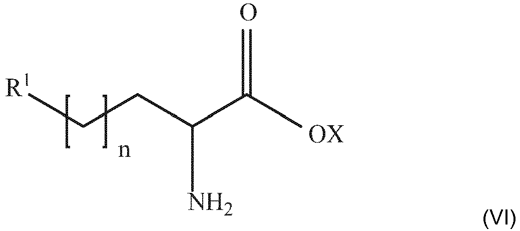 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
   c) weiterhin mindestens ein Alkalisierungsmittel, ausgewählt aus Ammoniumhydroxid, Monoethanolamin und Natriumsilikaten sowie Mischungen hiervon,
   d) ggf. mindestens ein Oxidationsfarbstoffvorprodukt und/oder mindestens einen direktziehenden Farbstoff,
   e) Wasser und
   f) mindestens eine Peroxidverbindung enthält.
      Ein weiterer Gegenstand der vorliegenden Erfindung ist ein Verfahren zur oxidativen Färbung und/oder Aufhellung keratinischer Fasern, insbesondere von Humanhaar, das folgende Verfahrensschritte umfasst:
      I. Bereitstellen einer Zusammensetzung (A), enthaltend
         a) mindestens eine gesättigte Dicarbonsäure mit 2 bis 10 Kohlenstoffatomen und/oder mindestens ein Salz dieser Säure(n),
         b) mindestens eine Aminosäure der Formel (VI) 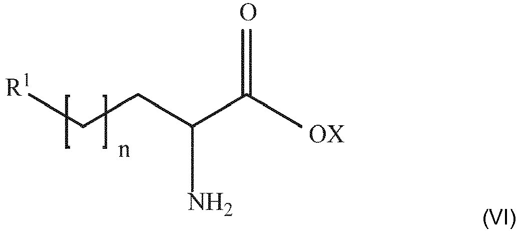 worin
            X für ein Wasserstoffatom oder ein ein- oder zweiwertiges Kation steht; n für null, 1, 2 oder 3 steht;
            R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe - SCH₃, oder mindestens ein Salz dieser Aminosäure,
         c) Wasser und
         d) optional weiterhin mindestens eine Substanz, die ausgewählt ist aus
            - Verbindungen der allgemeinen Formel (III), 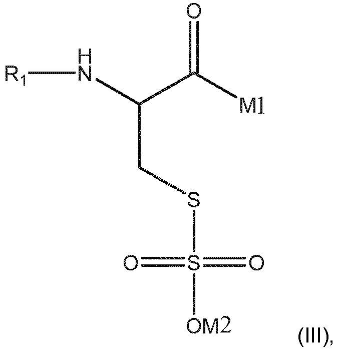 wobei
               R1 für ein Wasserstoffatom oder für ein Strukturelement der Formel (IV) steht 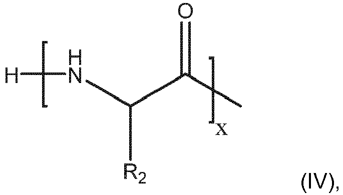 wobei
               x für eine ganze Zahl von 1 bis 100 steht, der Rest R2 in jedem der Strukturelemente der Formel (IV) jeweils unabhängig vom vorangegangenen Strukturelement der Formel (IV) gewählt werden kann,
               R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
               M1 steht für die Gruppierung -OM2 oder für ein Strukturelement der Formel (V) 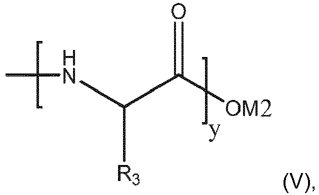 wobei
                  y für eine ganze Zahl von 1 bis 100 steht,
                  der Rest R3 in jedem der Strukturelemente der Formel (V) jeweils unabhängig vom vorangegangenen Strukturelement der Formel (V) gewählt werden kann, R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
                  M2 steht für ein Wasserstoffatom, ein Äquivalent eines ein oder mehrwertigen Kations oder ein Ammoniumion (NH₄)⁺, und
            - Polymeren A, die mindestens zehn konstitutive Einheiten der Formel (I) aufweisen, 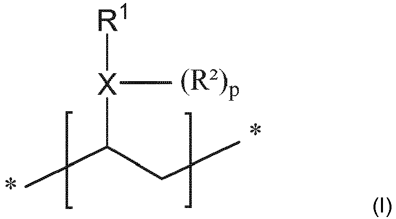 worin
               - X für Stickstoff oder Sauerstoff steht und
               - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 -C6-Alkylgruppe und/oder mit mindestens einer funktionellen Gruppe substituiert ist, und
               - p = 0 ist, wenn X für Sauerstoff steht und p = 1 ist, wenn X für Stickstoff steht, wobei das Polymer A keine permanent-ionischen konstitutiven Einheiten enthält,
      II. Bereitstellen einer Zusammensetzung (B), enthaltend
         e) mindestens ein Alkalisierungsmittel, ausgewählt aus Ammoniumhydroxid, Monoethanolamin und Natriumsilikaten sowie Mischungen hiervon,
         f) ggf. Wasser und
         g) ggf. mindestens ein Oxidationsfarbstoffvorprodukt und/oder mindestens einen direktziehenden Farbstoff,
      III. Bereitstellen einer Zusammensetzung (C), enthaltend
         h) mindestens eine Peroxidverbindung, die bevorzugt Wasserstoffperoxid ist,
   IV. Vermischen der Zusammensetzungen (A), (B) und (C) miteinander, direkt anschließend
   V. Auftragen der Mischung aus (A), (B) und (C) auf die keratinischen Fasern, insbesondere auf das Humanhaar, und
   VI. Ausspülen nach einer Einwirkzeit von 0,1 bis 60 Minuten, bevorzugt 1 bis 45 Minuten, besonders bevorzugt 10 bis 30 Minuten,
   VII. ggf. weitere Haarbehandlungen, wie Verformen, Konditionieren und/oder Trocknen.

Ein weiterer Gegenstand der vorliegenden Erfindung ist ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (AB), enthaltend
   a) mindestens eine gesättigte Dicarbonsäure mit 2 bis 10 Kohlenstoffatomen und/oder mindestens ein Salz dieser Säure(n),
   b) mindestens eine Aminosäure der Formel (VI), 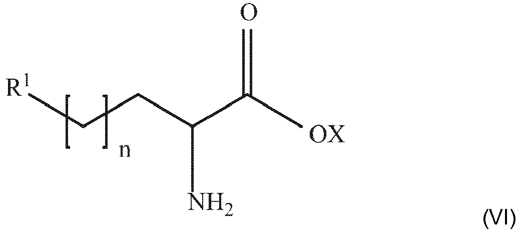 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
      wobei die Aminosäure der Formel (VI) bevorzugt ausgewählt ist aus Arginin, Lysin, Histidin sowie Mischungen hiervon, besonders bevorzugt Mischungen aus Arginin und Lysin, oder mindestens einem Salz dieser Aminosäuren,
   c) optional weiterhin mindestens eine Substanz, die ausgewählt ist aus
      - Verbindungen der allgemeinen Formel (III), 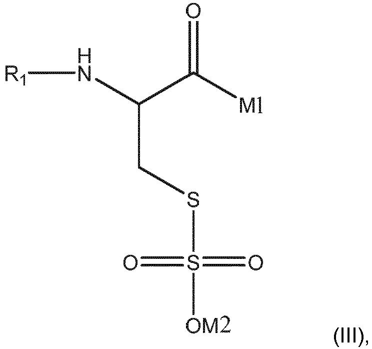 wobei
         R1 für ein Wasserstoffatom oder für ein Strukturelement der Formel (IV) steht 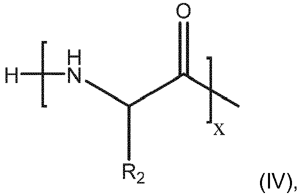 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann, R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
         M1 steht für die Gruppierung -OM2 oder für ein Strukturelement der Formel (V) 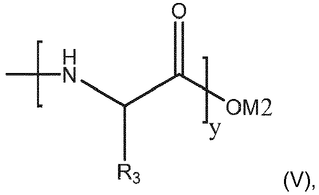 wobei
            y für eine ganze Zahl von 1 bis 100 steht, der Rest R3 in jedem der Strukturelemente der Formel (V) jeweils unabhängig vom vorangegangenen Strukturelement der Formel (V) gewählt werden kann,
            R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
            M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 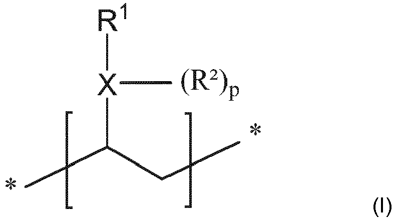 worin
         - X für Stickstoff oder Sauerstoff steht und
         - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
         - p = 0 ist, wenn X für Sauerstoff steht und p = 1 ist, wenn X für Stickstoff steht, wobei das Polymer A keine permanent-ionischen konstitutiven Einheiten enthält,
   d) mindestens ein Alkalisierungsmittel, ausgewählt aus Ammoniumhydroxid, Monoethanolamin und Natriumsilikaten sowie Mischungen hiervon,
   e) Wasser und
   f) ggf. mindestens ein Oxidationsfarbstoffvorprodukt und/oder mindestens einen direktziehenden Farbstoff,
II. Bereitstellen einer Zusammensetzung (C), enthaltend
   g) mindestens eine Peroxidverbindung, die bevorzugt Wasserstoffperoxid ist,
III. Vermischen der Zusammensetzungen (AB) und (C) miteinander, direkt anschließend
IV. Auftragen der Mischung aus (AB) und (C) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

### Gesättigte Dicarbonsäuren mit 2 bis 10 Kohlenstoffatomen und/oder mindestens ein Salz dieser Säure(n)

Erfindungsgemäß bevorzugte gesättigte Dicarbonsäuren mit 2 bis 10 Kohlenstoffatomen sind ausgewählt aus Bernsteinsäure, Äpfelsäure, Oxalsäure, Malonsäure, Adipinsäure, Pimelinsäure, Korksäure, Azelainsäure, Sebacinsäure, sowie Mischungen dieser Säuren. Erfindungsgemäß besonders bevorzugt sind Bernsteinsäure und Äpfelsäure. Erfindungsgemäß außerordentlich bevorzugt ist Bernsteinsäure, Die genannten Dicarbonsäuren leisten einen wesentlichen Beitrag zur verringerten Haarschädigung durch die erfindungsgemäßen Färbe- oder Blondiermittel.

Je nach pH-Wert des erfindungsgemäßen Färbe- oder Blondiermittels oder der in einem der erfindungsgemäßen Färbe- oder Blondierverfahren eingesetzten Zusammensetzungen (A) oder (AB) kann die mindestens eine gesättigte Dicarbonsäure mit 2 bis 10 Kohlenstoffatomen als undissoziierte Säure, teilweise dissoziiert oder vollständig dissoziiert vorliegen. Liegt die mindestens eine gesättigte Dicarbonsäure mit 2 bis 10 Kohlenstoffatomen teilweise dissoziiert oder vollständig dissoziiert vor, so ist das Gegenion ausgewählt aus physiologisch verträglichen Kationen, wie insbesondere den Alkalimetall-, Erdalkalimetall- und Zinkionen sowie Ammoniumionen, Alkylammonium-, Alkanolammonium- und Glucammoniumionen, insbesondere die Mono-, Di- und Trimethyl-, -ethyl- und -hydroxyethylammoniumionen. Ebenfalls bevorzugt sind die Salze der gesättigten Dicarbonsäuren mit 2 bis 10 Kohlenstoffatomen mit Amino-C₁-C₆-Alkanolen, insbesondere mit Monoethanolamin, und Amino-C₁-C₆-Alkandiolen, insbesondere mit 2-Amino-2-methylpropan-1-ol, 2-Amino-2-methylpropan-1,3-diol, 2-Aminopropan-1-ol, 3-Aminopropan-1-ol, 1-Aminopropan-2-ol (MIPA) und 2-Amino-2-(hydroxymethyl)propan-1,3-diol (TRIS), wobei die Salze mit Monoethanolamin, 2-Amino-2-methylpropan-1-ol und 2-Amino-2-methylpropan-1,3-diol besonders bevorzugt sind. Außerordentlich bevorzugt sind Natrium-, Kalium-, Magnesium-, Ammonium und Monoethanolammonium-lonen als Gegenionen für die teilweise oder vollständig dissoziierten gesättigten Dicarbonsäuren mit 2 bis 10 Kohlenstoffatomen. Daneben können jedoch auch mit alkalisch reagierenden Aminosäuren, wie beispielsweise Arginin, Lysin, Ornithin und Histidin, neutralisierte gesättigte Dicarbonsäuren mit 2 bis 10 Kohlenstoffatomen eingesetzt werden.

Die Natrium-, Kalium-, Ammonium-, Monoethanolammonium-, Lysin- sowie Argininsalze sowie deren Mischungen sind bevorzugte Salze der gesättigten Dicarbonsäuren mit 2 bis 10 Kohlenstoffatomen.

Bevorzugte erfindungsgemäße Färbe- oder Blondiermittel enthalten die mindestens eine gesättigte Dicarbonsäure mit 2 bis 10 Kohlenstoffatomen oder ein oder mehrere Salze hiervon in einer Gesamtmenge von 0,2 bis 4 Gew.-%, bevorzugt 0,33 bis 3 Gew.-%, besonders bevorzugt 0,5 bis 2 Gew.-%, jeweils umgerechnet auf die undissoziierte Dicarbonsäure und bezogen auf das Gewicht des Färbe- oder Blondiermittels.

Auch wenn die Dicarbonsäuren in Salzform vorliegen, beziehen sich die vorstehenden Mengenangaben auf die jeweilige Dicarbonsäure in undissoziierter Form, um die Mengenangabe nicht durch unterschiedliche Molgewichte der Salze zu verfälschen. Eine Einwaage von 15 Gew.-% Dinatriumsuccinathexahydrat ergäbe beispielsweise eine Konzentration an Bernsteinsäure von umgerechnet 6,55 Gew.-%.

### Aminosäure der Formel (VI)

Die verringerte Haarschädigungswirkung der erfindungsgemäßen Färbe- oder Blondiermittel ist wesentlich zurückzuführen auf die vorgenannten Dicarbonsäuren im Zusammenwirken mit mindestens einer ausgewählten Aminosäure der Formel (VI).

Die erfindungsgemäßen Färbe- oder Blondiermittel enthalten daher als weitere obligatorische Komponente mindestens eine Aminosäure der Formel (VI) 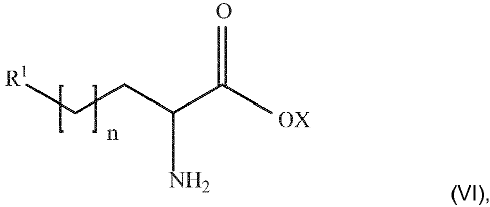 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens einem Salz dieser Aminosäure.

Bevorzugte Aminosäuren der Formel (VI) sind ausgewählt aus Arginin, Lysin, Histidin, Asparagin, Glutamin, Cystein, Methionin, Tryptophan sowie Mischungen hiervon. Besonders bevorzugte Färbe- oder Blondiermittel enthalten Mischungen aus Arginin und Lysin oder mindestens einem Salz dieser Aminosäuren.

Bevorzugte erfindungsgemäße Färbe- oder Blondiermittel enthalten die mindestens eine Aminosäure der Formel (VI) oder ein oder mehrere Salze hiervon in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Aminosäure und bezogen auf das Gewicht des Färbe- oder Blondiermittels. Weitere besonders bevorzugte erfindungsgemäße Färbe- oder Blondiermittel enthalten Mischungen aus Arginin und Lysin oder mindestens einem Salz dieser Aminosäuren in einer Gesamtmenge von 0,05 bis 3 Gew.-%, bevorzugt 0,1 bis 2 Gew.-%, besonders bevorzugt 0,2 bis 1,2 Gew.-%, jeweils umgerechnet auf die undissoziierte Säure und bezogen auf das Gewicht des Färbe- oder Blondiermittels.

### Alkalisierungsmittel

Die erfindungsgemäßen Färbe- oder Blondiermittel enthalten weiterhin mindestens ein Alkalisierungsmittel, ausgewählt aus Ammoniumhydroxid, Monoethanolamin und Natriumsilikaten sowie Mischungen hiervon.

Zur Erzielung der gewünschten haltbaren Färbung oder Aufhellung der keratinischen Fasern muss das erfindungsgemäße Färbe- oder Blondiermittel einen pH-Wert im Bereich von 6,5 bis 11,0, bevorzugt 8 bis 10,5, besonders bevorzugt 8,5 bis 10, jeweils gemessen bei 20°C, aufweisen. Bei diesen pH-Werten öffnet sich die äußere Keratinfaserschicht optimal zur Aufnahme der Oxidationsfarbstoffvorprodukte und entfaltet sich die gewünschte Wirkung der Peroxidverbindung optimal.

Bevorzugt wird Ammoniak in Form seiner wässrigen Lösung eingesetzt. Bei entsprechenden wässrigen Ammoniak-Lösungen kann es sich um 10 bis 35 prozentige Lösungen handeln (berechnet in Gew.-%, 100 g wässrige Ammoniaklösung enthalten dementsprechend 10 bis 35 g Ammoniak). Bevorzugt wird Ammoniak in Form einer 20 bis 30 Gew.-%igen Lösung, besonders bevorzugt in Form einer 25 Gew.-%igen Lösung eingesetzt.

In einer besonders bevorzugten Ausführungsform ist das erfindungsgemäße Färbe- oder Blondiermittel dadurch gekennzeichnet, dass es Ammoniumhydroxid in einer Menge von 0,20 bis 2,5 Gew.-%, bevorzugt von 0,5 bis 2,0 Gew.-%, weiter bevorzugt von 1,0 bis 1,5 Gew.-% und besonders bevorzugt von 0,31 bis 0,8 Gew.-% - bezogen auf das Gesamtgewicht des erfindungsgemäßen Färbe- oder Blondiermittels - enthält.

Zusätzlich zu bzw. anstelle von Ammoniumhydroxid enthalten bevorzugte erfindungsgemäße Färbe- oder Blondiermittel Monoethanolamin

Zur Erzielung einer maximalen Geruchsabdeckung und zur Optimierung der Echtheitseigenschaften ist Monoethanolamin in einer Gesamtmenge von 0,2 - 6,5 Gew.-%, bevorzugt von 0,5 - 4,0 Gew.-%, weiter bevorzugt von 0,7 bis 2,5 Gew.-% und besonders bevorzugt von 0,8 bis 1,6 Gew.-% - bezogen auf das Gesamtgewicht des erfindungsgemäßen Färbe- oder Blondiermittels - enthalten. Bei Natriumsilikaten im Sinne der vorliegenden Erfindung handelt es sich um chemische Verbindungen, die sich aus Natriumoxid und Siliciumdioxid zusammengesetzen und die in verschiedenen molaren Verhältnissen (Monosilikat, Metasilikat und Polysilikat) vorkommen können. Ein Beispiel für ein Natriumsilikat ist das Natriumsalz der Orthokieselsäure mit der Summenformel Na₄SiO₄, das auch als Natriumorthosilikat bezeichnet wird.

Weitere Beispiele für geeignete Natriumsilikate sind das Dinatriummetasilikat bzw. Natriummetasilikat mit der Summenformel Na₂SiO₃, das Dinatriumdisilikat mit der Summenformel Na₂Si₂O₅ oder das Dinatriumtrisilikat mit der Summenformel Na₂Si₃O₇.

Silikate in amorpher Form können durch das Zusammenschmelzen von Siliciumdioxid und Alkalioxid in molaren Verhältnissen zwischen 1:1 und 4:1 hergestellt werden. Die so erhaltenen Feststoffe werden bei etwa 150 °C und 5 bar Dampfdruck gelöst, um eine Lösung der Natriumsilikate in Wasser zu erhalten, bei diesen entsprechenden Lösungen handelt es sich um Alkaliwassergläser.

Als Alkaliwassergläser werden aus einer Schmelze erstarrte, glasartige (amorphe) Natriumsilikate oder ihre wässrigen Lösungen bezeichnet. Diese nennt man auch Natronwasserglas. Auch Natronwassergläser sind innerhalb dieser Erfindung von der Definition der Natriumsilikate umfasst.

Die molare Zusammensetzung bei Wassergläsern beträgt üblicherweise 2 bis 4 mol SiO₂ auf 1 mol Alkalioxid (Na₂O).

Ein Beispiel für ein bevorzugtes Natriumsilikat ist Natronwasserglas, das in Form seiner wässrigen Lösung vorliegt, einen Na₂O-Gehalt von 7,5 bis 8,8 Gew.-% und einen SiO2 Gehalt von 25,0 bis 28,5 Gew.-% besitzt und das die CAS-Nr. 1344-09-5 (Chemical Abstracts Number) hat.

Weitere erfindungsgemäß bevorzugte Färbe- oder Blondiermittel enthalten mindestens ein Natriumsilikat in einer Gesamtmenge von 0,1 bis 9 Gew.-%, bevorzugt 0,2 bis 8 Gew.-%, besonders bevorzugt 1 bis 7,5 Gew.-%, jeweils bezogen auf das Gesamtgewicht des erfindungsgemäßen Färbe- oder Blondiermittels.

Weiterhin können andere Alkalisierungsmittel, wie Kaliumhydroxid (KOH) und Natriumhydroxid (NaOH) enthalten sein, meist in einer Gesamtmenge von 0,05 bis 1,5 Gew.-%, bevorzugt 0,1 bis 0,6 Gew.-%, jeweils bezogen auf das Gesamtgewicht des erfindungsgemäßen Färbe- oder Blondiermittels.

Weiterhin wurde überraschend festgestellt, dass die verringerte Haarschädigungswirkung der erfindungsgemäßen und erfindungsgemäß bevorzugten Färbe- oder Blondiermittel weiter unterstützt werden kann, wenn mindestens eine Verbindung der allgemeinen Formel (III) enthalten ist. Erfindungsgemäß bevorzugte Färbe- oder Blondiermittel enthalten daher
(a) mindestens eine Verbindung der allgemeinen Formel (III) 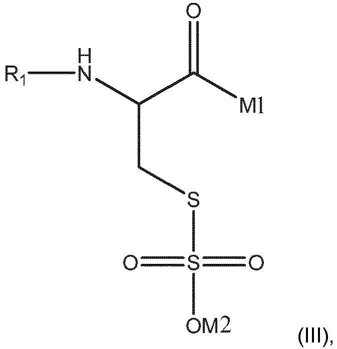 wobei
   R1 für ein Wasserstoffatom oder für ein Strukturelement der Formel (IV) steht 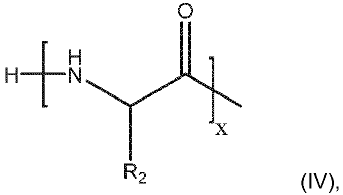 wobei
   x für eine ganze Zahl von 1 bis 100 steht,
   der Rest R2 in jedem der Strukturelemente der Formel (IV) jeweils unabhängig vom vorangegangenen Strukturelement der Formel (IV) gewählt werden kann,
   R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M1 steht für die Gruppierung -OM2 oder für ein Strukturelement der Formel (V) 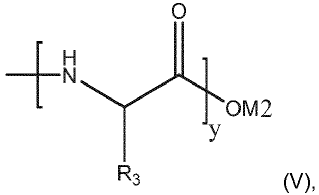 wobei
      y für eine ganze Zahl von 1 bis 100 steht,
      der Rest R3 in jedem der Strukturelemente der Formel (V) jeweils unabhängig vom vorangegangenen Strukturelement der Formel (V) gewählt werden kann,
      R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
      M2 steht für ein Wasserstoffatom, ein Äquivalent eines ein oder mehrwertigen Kations oder ein Ammoniumion (NH₄)⁺.

Bei dem wesentlichen Inhaltsstoff (a) der Formel (III) handelt es sich um das Bunte-Salz einer Aminosäure, eines Oligopeptids oder eines Peptids, das eine Verbindung der Formel (III) darstellt, 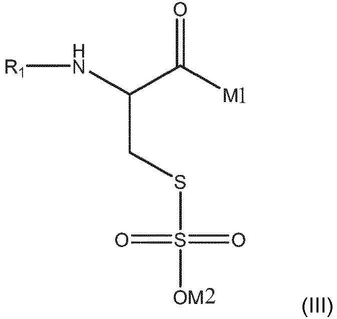 wobei
R1 für ein Wasserstoffatom oder für ein Strukturelement der Formel (IV) steht 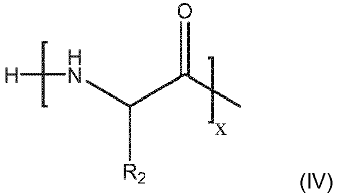 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 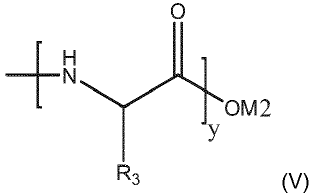 wobei
   y für eine ganze Zahl von 1 bis 100 steht
   der Rest R3 in jedem der Strukturelemente der Formel (V) jeweils unabhängig vom vorangegangenen Strukturelement der Formel (V) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder ein Ammoniumion (NH₄)⁺.

Der Rest R1 kann entweder für ein Wasserstoffatom oder für ein Strukturelement der Formel (IV) stehen

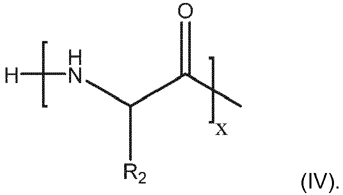

Das Strukturelement der Formel (IV) ist weiterhin gekennzeichnet durch den Wiederholungsindex x, wobei x für eine ganze Zahl von 1 bis 100 steht. Der Wiederholungsindex x gibt an, wie viele Strukturelemente der Formel (IV) in der Verbindung der Formel (III) enthalten sind.

Bevorzugt steht x für eine ganze Zahl von 1 bis 50, weiter bevorzugt steht x für eine ganze Zahl von 1 bis 20, und ganz besonders bevorzugt steht x für eine ganze Zahl von 1 bis 10.

Wenn x beispielweise für die Zahl 10 steht, beinhaltet die Verbindung der Formel (III) 10 Strukturelemente der Formel (IV).

Hierbei ist wesentlich, dass der Rest R2 in jedem der Strukturelemente der Formel (IV) jeweils unabhängig vom vorangegangenen Strukturelement der Formel (IV) gewählt werden kann. Enthalten die Verbindungen der Formel (III) beispielsweise 10 Struktureinheiten der Formel (IV), so können diese 10 Struktureinheiten gleich oder aber auch verschieden sein.

Der Rest R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)-ethyl-Gruppe, eine 1H-Imidazol-4-ylmethyl-Gruppe, eine 1H-Indol-3-ylmethyl-Gruppe oder für eine (Sulfosulfanyl)methyl-Gruppe.

Bei dem Strukturelement der Formel (IV) handelt es sich somit um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R2 für ein Wasserstoffatom steht, so beruht das Strukturelement der Formel (IV) auf der Aminosäure Glycin.

Wenn der Rest R2 für eine Methylgruppe steht, so beruht das Strukturelement der Formel (IV) auf der Aminosäure Alanin.

Wenn der Rest R2 für eine Isopropylgruppe (d.h. eine Gruppe (H₃C)₂CH-) steht, so beruht das Strukturelement der Formel (IV) auf der Aminosäure Valin.

Wenn der Rest R2 für eine 2-Methylpropylgruppe (d.h. eine Gruppe (H₃C)₂CH-CH₂-), so beruht das Strukturelement der Formel (IV) auf der Aminosäure Leucin.

Wenn der Rest R2 für eine 1-Methyl-propylgruppe (d.h. eine Gruppe H3C-CH2-CH(CH3)-) steht, so beruht das Strukturelement der Formel (IV) auf der Aminosäure Isoleucin.

Wenn der Rest R2 für eine Benzylgruppe (d.h. eine Gruppe C₆H₅-CH₂-) steht, so beruht das Strukturelement der Formel (IV) auf der Aminosäure Phenylalanin.

Wenn der Rest R2 für eine 4-Hydroxybenzylgruppe (d.h. eine Gruppe 4-OH-C₆H₅-CH₂-) steht, so beruht das Strukturelement der Formel (IV) auf der Aminosäure Tyrosin.

Wenn der Rest R2 für eine Hydroxymethylgruppe (d.h. eine Gruppe HO-CH2-) steht, so beruht das Strukturelement der Formel (IV) auf der Aminosäure Serin.

Wenn der Rest R2 für eine 1-Hydroxyethylgruppe (d.h. eine Gruppe H3C-CH(OH)-) steht, so beruht das Strukturelement der Formel (IV) auf der Aminosäure Threonin.

Wenn der Rest R2 für eine 4-Aminobutylgruppe (d.h. eine Gruppe H2N-CH2-CH2-CH2-CH2-) steht, so beruht das Strukturelement der Formel (IV) auf der Aminosäure Lysin.

Wenn der Rest R2 für eine 3-Carbamimidamidopropyl-Gruppe (d.h. eine Gruppe H₂N-C(NH)-NH-CH₂-CH₂-CH₂-) steht, so beruht das Strukturelement der Formel (IV) auf der Aminosäure Arginin. Wenn der Rest R2 für eine 2-Carboxyethyl-Gruppe (d.h. eine Gruppe HOOC-CH2-CH2-) steht, so beruht das Strukturelement der Formel (IV) auf der Aminosäure Glutaminsäure.

Wenn der Rest R2 für eine Carboxymethyl-Gruppe (d.h. eine Gruppe HOOC-CH2-) steht, so beruht das Strukturelement der Formel (IV) auf der Aminosäure Asparaginsäure.

Wenn der Rest R2 für eine 2-Carbamoylethyl-Gruppe (d.h. eine Gruppe H2N-C(O)-CH2-CH2-) steht, so beruht das Strukturelement der Formel (IV) auf der Aminosäure Glutamin.

Wenn der Rest R2 für eine Carbamoylmethyl-Gruppe (d.h. eine Gruppe H2N-C(O)-CH2-) steht, so beruht das Strukturelement der Formel (IV) auf der Aminosäure Asparagin.

Wenn der Rest R2 für eine Sulfanylmethyl-Gruppe (d.h. eine Gruppe HS-CH2-) steht, so beruht das Strukturelement der Formel (IV) auf der Aminosäure Cystein.

Wenn der Rest R2 für eine 2-(Methylsulfanyl)ethyl-Gruppe (d.h. eine Gruppe H3C-S-CH2-CH2-) steht, so beruht das Strukturelement der Formel (IV) auf der Aminosäure Methionin.

Wenn der Rest R2 für eine 1H-Imidazol-4-ylmethyl-Gruppe steht, so beruht das Strukturelement der Formel (IV) auf der Aminosäure Histidin.

Wenn der Rest R2 für eine 1H-Indol-3-ylmethyl-Gruppe steht, so beruht das Strukturelement der Formel (IV) auf der Aminosäure Tryptophan.

Schließlich kann der Rest R2 auch für eine (Sulfosulfanyl)methyl-Gruppe stehen, hierbei handelt es sich um eine Bunte-Salz-Struktur der Formel HO-S(O₂)-S-CH₂-.

Je nach pH-Wert des Färbe- oder Blondiermittels kann die Bunte-Salz-Struktur der Formel HO-S(O₂)-S-CH₂- auch in ihrer deprotonierten Form vorliegen.

Innerhalb der Verbindung der Formel (III) steht M1 steht die Gruppierung -OM2 oder für ein Strukturelement der Formel (V)

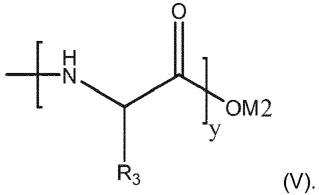

Das Strukturelement der Formel (V) ist gekennzeichnet durch den Wiederholungsindex y, wobei y für eine ganze Zahl von 1 bis 100 steht. Der Wiederholungsindex y gibt an, wie viele Strukturelemente der Formel (V) in der Verbindung der Formel (III) enthalten sind.

Bevorzugt steht y für eine ganze Zahl von 1 bis 50, weiter bevorzugt steht y für eine ganze Zahl von 1 bis 20, und ganz besonders bevorzugt steht y für eine ganze Zahl von 1 bis 10.

Wenn y beispielweise für die Zahl 10 steht, beinhaltet die Verbindung der Formel (III) 10 Strukturelemente der Formel (V).

Hierbei ist wesentlich, dass der Rest R3 in jedem der Strukturelemente der Formel (V) jeweils unabhängig vom vorangegangenen Strukturelement der Formel (V) gewählt werden kann. Enthalten die Verbindungen der Formel (III) beispielsweise 10 Struktureinheiten der Formel (V), so können diese 10 Struktureinheiten gleich oder aber auch verschieden sein.

Der Rest R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)-ethyl-Gruppe, eine 1H-Imidazol-4-ylmethyl-Gruppe, eine 1H-Indol-3-ylmethyl-Gruppe oder eine (Sulfosulfanyl)methyl-Gruppe.

Damit handelt es sich auch bei dem Strukturelement der Formel (V) um eine Aminosäure, die peptidisch über ihre Amino- und/oder ihre Säurefunktion innerhalb der Verbindung der Formel (III) verknüpft ist. Wenn es sich bei der Aminosäure um Cystein handelt, kann diese auch in Form eines Bunte-Salzes vorliegen.

Wenn der Rest R3 für ein Wasserstoffatom steht, so beruht das Strukturelement der Formel (V) auf der Aminosäure Glycin.

Wenn der Rest R3 für eine Methylgruppe steht, so beruht das Strukturelement der Formel (V) auf der Aminosäure Alanin.

Wenn der Rest R3 für eine Isopropylgruppe (d.h. eine Gruppe (H₃C)₂CH-) steht, so beruht das Strukturelement der Formel (V) auf der Aminosäure Valin.

Wenn der Rest R3 für eine 2-Methylpropylgruppe (d.h. eine Gruppe (H₃C)₂CH-CH₂-), so beruht das Strukturelement der Formel (V) auf der Aminosäure Leucin.

Wenn der Rest R3 für eine 1-Methyl-propylgruppe (d.h. eine Gruppe H3C-CH2-CH(CH3)-) steht, so beruht das Strukturelement der Formel (V) auf der Aminosäure Isoleucin.

Wenn der Rest R3 für eine Benzylgruppe (d.h. eine Gruppe C₆H₅-CH₂-) steht, so beruht das Strukturelement der Formel (V) auf der Aminosäure Phenylalanin.

Wenn der Rest R3 für eine 4-Hydroxybenzylgruppe (d.h. eine Gruppe 4OH-C₆H₅-CH₂-) steht, so beruht das Strukturelement der Formel (V) auf der Aminosäure Tyrosin.

Wenn der Rest R3 für eine Hydroxymethylgruppe (d.h. eine Gruppe HO-CH2-) steht, so beruht das Strukturelement der Formel (V) auf der Aminosäure Serin.

Wenn der Rest R3 für eine 1-Hydroxyethylgruppe (d.h. eine Gruppe H3C-CH(OH)-) steht, so beruht das Strukturelement der Formel (V) auf der Aminosäure Threonin.

Wenn der Rest R3 für eine 4-Aminobutylgruppe (d.h. eine Gruppe H2N-CH2-CH2-CH2-CH2-) steht, so beruht das Strukturelement der Formel (V) auf der Aminosäure Lysin.

Wenn der Rest R3 für eine 3-Carbamimidamidopropyl-Gruppe (d.h. eine Gruppe H₂N-C(NH)-NH-CH₂-CH₂-CH₂-) steht, so beruht das Strukturelement der Formel (V) auf der Aminosäure Arginin. Wenn der Rest R3 für eine 2-Carboxyethyl-Gruppe (d.h. eine Gruppe HOOC-CH2-CH2-) steht, so beruht das Strukturelement der Formel (V) auf der Aminosäure Glutaminsäure.

Wenn der Rest R3 für eine Carboxymethyl-Gruppe (d.h. eine Gruppe HOOC-CH2-) steht, so beruht das Strukturelement der Formel (V) auf der Aminosäure Asparaginsäure.

Wenn der Rest R3 für eine 2-Carbamoylethyl-Gruppe (d.h. eine Gruppe H2N-C(O)-CH2-CH2-) steht, so beruht das Strukturelement der Formel (V) auf der Aminosäure Glutamin.

Wenn der Rest R3 für eine Carbamoylmethyl-Gruppe (d.h. eine Gruppe H2N-C(O)-CH2-) steht, so beruht das Strukturelement der Formel (V) auf der Aminosäure Asparagin.

Wenn der Rest R3 für eine Sulfanylmethyl-Gruppe (d.h. eine Gruppe HS-CH2-) steht, so beruht das Strukturelement der Formel (V) auf der Aminosäure Cystein.

Wenn der Rest R3 für eine 2-(Methylsulfanyl)ethyl-Gruppe (d.h. eine Gruppe H3C-S-CH2-CH2-) steht, so beruht das Strukturelement der Formel (V) auf der Aminosäure Methionin.

Wenn der Rest R3 für eine 1H-Imidazol-4-ylmethyl-Gruppe steht, so beruht das Strukturelement der Formel (V) auf der Aminosäure Histidin.

Wenn der Rest R3 für eine 1H-Indol-3-ylmethyl-Gruppe steht, so beruht das Strukturelement der Formel (V) auf der Aminosäure Tryptophan.

Schließlich kann der Rest R3 auch für eine (Sulfosulfanyl)methyl-Gruppe stehen, hierbei handelt es sich um eine Bunte-Salz Struktur der Formel HO-S(O₂)-S-CH₂-.

Je nach pH-Wert des Färbe- oder Blondiermittels kann auch hier die Bunte-Salz Struktur der Formel HO-S(O₂)-S-CH₂- auch in ihrer deprotonierten Form vorliegen.

Der Rest M2 steht für ein Wasserstoffatom, ein Äquivalent eines ein oder mehrwertigen Kations oder ein Ammoniumion (NH₄)⁺.

Als bevorzugte Äquivalente eines ein oder mehrwertigen Kations können insbesondere die Kationen von Natrium und Kalium (Na⁺ bzw. K⁺) oder auch Magnesium oder Calcium (1/2 Mg²⁺ oder ½ Ca²⁺) genannt werden.

Steht M2 für ein Wasserstoffatom, so handelt es sich bei der Gruppierung -OM2 um die Gruppierung -OH. Steht M2 für ein Natriumkation, so handelt es sich bei der Gruppierung -OM2 um die Gruppierung -ONa. Steht M2 für ein Kaliumkation, so handelt es sich bei der Gruppierung -OM2 um die Gruppierung -OK. Steht M2 für ein Ammoniumion, so handelt es sich bei der Gruppierung -OM2 um die Gruppierung -O(NH₄).

Die Gruppierung -OM2 ist stets einer Carbonylgruppe benachbart. In Summe liegt, wenn M2 für H, K, Na oder Ammonium steht, damit in der Verbindung der Formel (III) daher entweder in Form eine Säure in ihrer protonierten Form oder aber das Natrium, Kalium oder Ammoniumsalz dieser Säure vor.

Bei den erfindungsgemäßen Verbindungen der Formel (III) handelt es sich entweder um das Bunte-Salz der Aminosäure Cystein, um die Bunte-Salze von Oligo-Peptiden oder aber um die Bunte-Salze von Peptiden.

Wenn der Rest R1 für ein Wasserstoffatom steht und der Rest M1 für eine Gruppierung -OM2 steht, dann handelt es sich bei der Verbindung der Formel (III) um das Bunte-Salz der Aminosäure Cystein. In diesem Fall handelt es sich bei der Verbindung der Formel (III) um die Verbindung der Formel (lila), 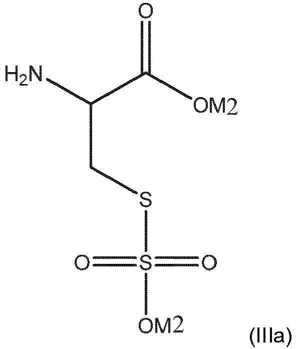 wobei M2 wieder wie zuvor beschrieben definiert.

Liegt die Verbindung der Formel (IIIa) in Form ihrer freien Säure vor, so handelt es sich um 2-Amino-3-(sulfosulfanyl)propansäure. Diese Substanz ist kommerziell erhältlich.

Es hat sich herausgestellt, dass der Einsatz der Verbindung der Formel (IIIa) in Färbe- oder Blondiermittel bereits bei besonders geringen Einsatzmengen zu einer besonders effektiven Reduzierung der Haarschädigung führt, die auch nach wiederholten Haarwäschen noch vorhanden ist. Deshalb ist der Einsatz von Verbindungen der Formel (IIIa) in Färbe- oder Blondiermitteln ganz besonders bevorzugt.

In einer ganz besonders bevorzugten Ausführungsform ist ein erfindungsgemäßes Färbe- oder Blondiermittel, dadurch gekennzeichnet, dass es mindestens eine Verbindung der Formel (III) enthält, wobei
R1 für ein Wasserstoffatom steht und
M1 für eine Gruppierung -OM2 steht.

Wenn eine Verbindung der Formel (IIIa) eingesetzt wird, so handelt es sich bevorzugt um den Einsatz dieser spezifischen Verbindung. Werden als Verbindungen der Formel (III) jedoch die Bunte-Salze von Oligopeptiden eingesetzt, so kann das erfindungsgemäße Färbe- oder Blondiermittel auch mehrere Verbindungen der Formel (III) als Gemisch verschiedener Oligopeptide enthalten. Diese Oligopeptide werden durch ihr mittleres Molgewicht definiert. Das mittlere Molekulargewicht M_{w} des mindestens einen Oligopeptids der Formel (III) kann beispielsweise durch Gelpermeationschromatographie (GPC) mit Polystyrol als internem Standard gemäß DIN 55672-3, Version 8/2007, bestimmt werden.

Je nachdem, wie viele Strukturelemente der Formel (IV) und/oder (V) in der Verbindung der Formel (III) enthält sind, und je nach Art dieser Aminosäuren kann das Molgewicht der erfindungsgemäß verwendeten Verbindung der Formel (III) variieren. Es ist erfindungsgemäß besonders bevorzugt, wenn es sich bei der Verbindung der Formel (III) um ein Oligopeptid handelt, das ein Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Unter dem Begriff "Oligopeptid" im Rahmen der vorliegenden Erfindung werden Kondensationsprodukte von Aminosäuren verstanden, welche die oben genannten Molekulargewichte besitzen.

In einer ganz besonders bevorzugten Ausführungsform ist ein erfindungsgemäßes Färbe- oder Blondiermittel dadurch gekennzeichnet, dass es mindestens eine Verbindung der Formel (III) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird im erfindungsgemäßen Färbe- oder Blondiermittel eine Mischung von Oligomeren eingesetzt, so können diese Mischungen durch ihr mittleres Molekulargewicht definiert werden.

In diesem Fall ist ein bevorzugtes erfindungsgemäßes Färbe- oder Blondiermittel dadurch gekennzeichnet, dass es mindestens eine Mischung von Verbindungen der Formel (III) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (III) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (III) nimmt auch der Anteil der Bunte-Salz Einheiten, die in der Verbindung der Formel (III) enthalten sind, einen entscheidenden Einfluss auf die Wirksamkeit der Schutzwirkung oder "Repair-Wirkung" der Verbindungen.

Verbindungen mit mindestens einer Bunte-Salz-Einheit - wie sie beispielsweise in der Verbindung der Formel (IIIa) vorhanden ist-sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (III) mindestens zwei, bevorzugt mindestens drei Bunte-Salz Einheiten besitzt.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
der Rest R2 in mindestens einem Strukturelement der Formel (IV) für eine (Sulfosulfanyl)methyl-Gruppe (d.h. eine Gruppe HO-S(O₂)-S-CH₂-) steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist ein erfindungsgemäßes Färbe- oder Blondiermittel dadurch gekennzeichnet, dass es mindestens eine Verbindung der Formel (III) enthält, wobei
M1 für ein Strukturelement der Formel (V) steht, und
y für eine ganze Zahl von mindestens 3 steht und
der Rest R3 in mindestens 3 Strukturelementen der Formel (IV) für eine Gruppe (Glu) steht. Die mindestens eine Verbindung der Formel (III) ist - bezogen auf das Gesamtgewicht des erfindungsgemäß bevorzugten Färbe- oder Blondiermittels in einer Gesamtmenge von 0,001 bis 10 Gew.-% enthalten. Überraschenderweise hat sich jedoch herausgestellt, dass die Verbindung(en) der Formel (III) bereits in geringen Einsatzkonzentrationen eine sehr gute Reduzierung der Haarschädigung bewirken können. Dies ist insbesondere dann von Vorteil, wenn die mindestens eine Verbindung der Formel (III) dem erfindungsgemäßen Färbe- oder Blondiermittel als Additiv (beispielsweise in Form einer Pflegelösung oder Reparaturlösung) vor der Applikation auf das Haar hinzugefügt werden sollen. Aus diesem Grund ist es besonders vorteilhaft, wenn das erfindungsgemäß bevorzugte Färbe- oder Blondiermittel eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbe- oder Blondiermittels.

In einer weiteren ganz besonders bevorzugten Ausführungsform ist ein erfindungsgemäßes Färbe- oder Blondiermittel dadurch gekennzeichnet, dass es eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht des erfindungsgemäßen Färbe- oder Blondiermittels.

### Wasser

Die erfindungsgemäßen Färbe- oder Blondiermittel enthalten Wasser, und zwar bevorzugt in einer Menge von 20 bis 85 Gew.-%, bevorzugt 30 bis 80 Gew.-%, jeweils bezogen auf das Gesamtgewicht des erfindungsgemäßen Färbe- oder Blondiermittels.

### Peroxidverbindungen

Die Ausbildung der Farbstoffe in oxidativen Färbemitteln bzw. der Abbau des haareigenen Farbstoffs Melanin zur Blondierung erfolgt erst durch den Einfluss einer Peroxidverbindung als Oxidationsmittel. Üblicherweise wird hierfür Wasserstoffperoxid verwendet. Der Einsatz von Wasserstoffperoxid kann nur in Form einer wässrigen Lösung erfolgen.

Erfindungsgemäß bevorzugte Färbe- oder Blondiermittel sind dadurch gekennzeichnet, dass sie 0,5 bis 13 Gew.-%, weiter bevorzugt 1 bis 7 Gew.-%, besonders bevorzugt 2 bis 6 Gew.-% und ganz besonders bevorzugt 3 bis 4,5 Gew.-% Wasserstoffperoxid (berechnet als 100 %-iges H₂O₂) enthalten, jeweils bezogen auf das Gesamtgewicht des erfindungsgemäßen Färbe- oder Blondiermittels. Blondiermittel oder besonders stark aufhellende Färbemittel können darüber hinaus stark oxidierende Peroxidverbindungen, wie Kalium-, Natrium- und/oder Ammoniumpersulfat, enthalten.

Es hat sich als vorteilhaft erwiesen, wenn die erfindungsgemäßen Oxidationsmittelzubereitungen zur Stabilisierung des Wasserstoffperoxids zusätzlich mindestens einen Stabilisator oder Komplexbildner enthalten. Besonders bevorzugte Stabilisatoren sind insbesondere EDTA und EDDS, und Phosphonate, insbesondere 1-Hydroxyethan-1,1-diphosphonat (HEDP) und/oder Ethylendiamintetramethylenphosphonat (EDTMP) und/oder Diethylentriaminpentamethylenphosphonat (DTPMP) bzw. deren Natriumsalze.

Sofern es sich bei den erfindungsgemäßen Mitteln um Mittel zum oxidativen Färben von Keratinfasern, insbesondere Humanhaar, handelt, enthalten diese zur Ausbildung der Farbstoffe mindestens ein Oxidationsfarbstoffvorprodukt.

Unter die Oxidationsfarbstoffvorprodukte fallen Oxidationsfarbstoffvorprodukte vom Entwickler-Typ und vom Kuppler-Typ. Besonders geeignete Oxidationsfarbstoffvorprodukte vom Entwickler-Typ sind dabei ausgewählt aus mindestens einer Verbindung aus der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol, 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, 2,3-Diamino-6,7-dihydro-1H,5H-pyrazolo[1,2-a]pyrazol-1-on sowie deren physiologisch verträglichen Salzen.

Besonders geeignete Oxidationsfarbstoffvorprodukte vom Kuppler-Typ sind ausgewählt aus der Gruppe 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)ethanol, 1,3-Bis(2,4-diaminophenoxy)propan, 1-Methoxy-2-amino-4-(2-hydroxyethylamino)benzol, 1,3-Bis-(2,4-diaminophenyl)propan, 2,6-Bis(2'-hydroxyethylamino)-1-methylbenzol, 2-({3-[(2-Hydroxyethyl)-amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen.

In einer bevorzugten Ausführungsform enthalten die erfindungsgemäßen Färbemittel ein oder mehrere Oxidationsfarbstoffvorprodukte in einer Gesamtmenge von 0,01 bis 4,0 Gew.-%, bevorzugt von 0,1 bis 3,5 Gew.-%, weiter bevorzugt von 0,6 bis 3,1 Gew.-% und ganz besonders bevorzugt von 1,2 bis 2,2 Gew.-%, bezogen auf das Gesamtgewicht des erfindungsgemäßen Färbe- oder Blondiermittels.

In einer weiteren bevorzugten Ausführungsform enthalten die erfindungsgemäßen Mittel zusätzlich mindestens einen weiteren direktziehenden Farbstoff. Direktziehende Farbstoffe können in anionische, kationische und nichtionische direktziehende Farbstoffe unterteilt werden. Die direktziehenden Farbstoffe sind vorzugsweise ausgewählt aus den Nitrophenylendiaminen, den Nitroaminophenolen, den Azofarbstoffen, den Anthrachinonen, den Triarylmethanfarbstoffen oder den Indophenolen und deren physiologisch verträglichen Salzen. Die direktziehenden Farbstoffe sind jeweils bevorzugt in einer Gesamtmenge von 0,001 bis 2 Gew.-%, bezogen auf das Gesamtgewicht des erfindungsgemäßen Färbe- oder Blondiermittels, enthalten. Direktziehende Farbstoffe dienen in oxidativen Färbemitteln zur Nuancierung des erzielten Farbtons, in oxidativen Blondiermitteln zum Ausgleich unerwünschter Rottöne, die beim Abbau des haareigenen Melanins entstehen können. Bevorzugte anionische direktziehende Farbstoffe sind die unter den internationalen Bezeichnungen bzw. Handelsnamen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52, Bromphenolblau und Tetrabromphenolblau bekannten Verbindungen.

Bevorzugte kationische direktziehende Farbstoffe sind kationische Triphenylmethanfarbstoffe, wie beispielsweise Basic Blue 7, Basic Blue 26, Basic Violet 2 und Basic Violet 14, aromatische Systeme, die mit einer quaternären Stickstoffgruppe substituiert sind, wie beispielsweise Basic Yellow 57, Basic Red 76, Basic Blue 99, Basic Brown 16 und Basic Brown 17, kationische Anthrachinonfarbstoffe, wie HC Blue 16 (Bluequat B) sowie direktziehende Farbstoffe, die einen Heterocyclus enthalten, der mindestens ein quaternäres Stickstoffatom aufweist, insbesondere Basic Yellow 87, Basic Orange 31 und Basic Red 51. Die kationischen direktziehenden Farbstoffe, die unter dem Warenzeichen Arianor vertrieben werden, sind erfindungsgemäß ebenfalls bevorzugte kationische direktziehende Farbstoffe.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 7,HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9 bekannten Verbindungen, sowie 1,4-Diamino-2-nitrobenzol, 2-Amino-4-nitrophenol, 1,4-Bis-(2-hydroxyethyl)-amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 4-[(3-Hydroxypropyl)amino]-3-nitrophenol, 4-Nitro-o-phenylendiamin, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

### Optional: Polymer A mit mindestens 10 konstitutiven Einheiten der Formel (I)

Erfindungsgemäß bevorzugte Oxidationsfärbe- oder -blondiermittel enthalten optional mindestens ein Polymer A, das mindestens zehn konstitutive Einheiten der Formel (I) aufweist, 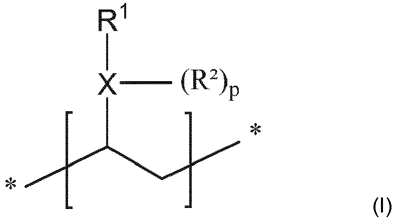 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält.

Überraschend wurde festgestellt, dass ein Polymer A, wie vorstehend bezeichnet und nachstehend näher erläutert, die Schutz- und Reparaturwirkung, die die Kombination aus mindestens einer gesättigten Dicarbonsäure mit 2 bis 10 Kohlenstoffatomen und/oder mindestens einem Salz dieser Säure(n) und mindestens einer Aminosäure der Formel (VI), wie vorstehend erläutert, auf oxidativ gefärbte oder blondierte keratinische Fasern ausübt, in hervorragender Weise unterstützt.

Unter "Polymer" werden im Sinne der vorliegenden Anmeldung Polymere im Sinne der IUPAC-Definition verstanden, die mindestens 10 identische konstitutive Einheiten umfassen.

Gemäß dem RÖMPP Chemie Lexikon, Stand Juli 2009, wird nach einer Definition der IUPAC eine Substanz als Polymer bezeichnet, die sich aus einem Kollektiv chemisch einheitlich aufgebauter Makromoleküle (Polymermoleküle) zusammensetzt, wobei sich diese Makromoleküle oder Polymermoleküle hinsichtlich Polymerisationsgrad, Molmasse und Kettenlänge voneinander unterscheiden. Bei derartigen so genannten polymereinheitlichen Stoffen sind also alle Makromoleküle gleich aufgebaut und unterscheiden sich lediglich durch ihre Kettenlänge (Polymerisationsgrad). Nach dieser IUPAC-Definition ist ein Polymer ferner "ein Polyreaktionsprodukt, das aus einer Vielzahl von Molekülen aufgebaut ist, in denen eine Art oder mehrere Arten von Atomen oder Atomgruppierungen (so genannte konstitutive Einheiten, Grundbausteine oder Wiederholungseinheiten) wiederholt aneinander gereiht sind.

Die Anzahl an konstitutiven Einheiten in einem Polymer bezeichnet man als Polymerisationsgrad. Erfindungsgemäß bevorzugte Polymere A wie auch Polymere B weisen jeweils einen Polymerisationsgrad im Bereich von 40 bis 1000, bevorzugt 100 bis 800, besonders bevorzugt 350 bis 650, auf. Weitere erfindungsgemäß bevorzugte Polymere A mit mindestens zehn konstitutiven Einheit der Formel (I) enthalten 40 bis 1000, bevorzugt 100 bis 800, besonders bevorzugt 350 bis 650 identische konstitutive Einheiten der Formel (I).

R¹ und R² stehen bevorzugt jeweils unabhängig voneinander für Wasserstoff oder für eine C₂-C₁₀-Acylgruppe, die bevorzugt ausgewählt aus einer Acetyl-, Propanoyl-, oder n-Butanoyl-Gruppe, besonders bevorzugt ausgewählt aus einer Acetyl-Gruppe.

Erfindungsgemäß bevorzugte Polymere A weisen mindestens 10 konstitutive Einheiten der Formel (I) auf, in denen X für Stickstoff steht, wobei das Polymer A keine permanent-ionischen konstitutiven Einheiten enthält.

Weitere erfindungsgemäß besonders bevorzugte Polymere A weisen mindestens 10 konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist.

Wenn R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, so ist dieser Ring bevorzugt mit mindestens einer funktionellen Gruppe substituiert, die ausgewählt ist aus =O. Ein besonders bevorzugte Substituenten-Kombination X, R¹, R² ist eine Pyrrolidon-Gruppe, so dass es sich bei einer erfindungsgemäß besonders bevorzugten konstitutiven Einheit der Formel (I) um eine Einheit der Formel (la) handelt, 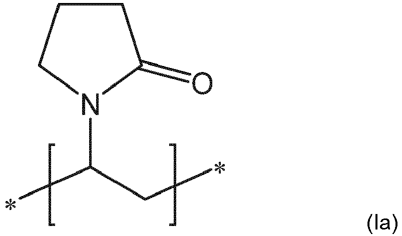 in der X für Stickstoff steht und R¹ und R² zusammen mit diesem Stickstoffatom einen fünfgliedrigen gesättigten Ring bilden, der keine weiteren Heteroatome enthält und der in 2-Position mit einer funktionellen Gruppe =O substituiert ist.

Eine weitere besonders bevorzugte Substituenten-Kombination X, R¹, R² ist eine ε-Caprolactam-Gruppe, so dass es sich bei einer erfindungsgemäß besonders bevorzugten konstitutiven Einheit der Formel (I) um eine Einheit der Formel (Ib) handelt, 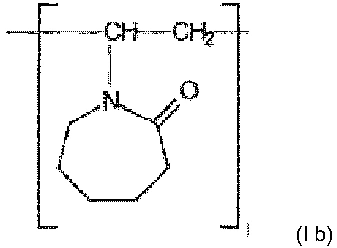 in der X für Stickstoff steht und R¹ und R² zusammen mit diesem Stickstoffatom einen sechsgliedrigen gesättigten Ring bilden, der keine weiteren Heteroatome enthält und der mit einer funktionellen Gruppe =O substituiert ist.

Eine weitere besonders bevorzugte Substituenten-Kombination X, R¹, R² ist eine Imidazol-Gruppe, so dass es sich bei einer weiteren erfindungsgemäß besonders bevorzugten Einheit der Formel (I) um eine Einheit handelt, in der X für Stickstoff steht und R¹ und R² zusammen mit diesem Stickstoffatom einen fünfgliedrigen ungesättigten Ring bilden, der als weiteres Heteroatom Stickstoff enthält. Weitere erfindungsgemäß bevorzugte Polymere A weisen zu 25 - 100 Mol-%, bevorzugt zu 55-100 Mol-%, besonders bevorzugt zu 85 - 100 Mol-% konstitutive Einheiten der Formel (I) auf, in denen X für Stickstoff steht, wobei das Polymer A keine permanent-ionischen konstitutiven Einheiten enthält.

Weitere erfindungsgemäß bevorzugte Polymere A weisen zu 25 - 100 Mol-%, bevorzugt zu 55-100 Mol-%, besonders bevorzugt zu 85 - 100 Mol-% konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die ausgewählt sind aus N und O und gegebenenfalls mit mindestens einer C1 - C6-Alkylgruppe und/oder mit mindestens einer funktionellen Gruppe substituiert ist, wobei das Polymer A keine permanent-ionischen konstitutiven Einheiten enthält.

Erfindungsgemäß besonders bevorzugte Polymere A weisen zu 98- 100 Mol-% konstitutive Einheiten der Formel (Ia) auf, wobei das Polymer A keine permanent-ionischen konstitutiven Einheiten enthält.

Erfindungsgemäß außerordentlich bevorzugte Polymere A weisen zu 98 - 100 Mol-% konstitutive Einheiten der Formel (Ia) auf und haben einen Polymerisationsgrad im Bereich von 40 bis 1000, bevorzugt 100 bis 800, besonders bevorzugt 350 bis 650, wobei das Polymer A keine permanent-ionischen konstitutiven Einheiten enthält. Besonders bevorzugte Polymere A sind Polyvinylpyrrolidon-Homopolymere mit einem Polymerisationsgrad im Bereich von 40 bis 1000, bevorzugt 100 bis 800, besonders bevorzugt 350 bis 650.

Eine weitere besonders bevorzugte Substituenten-Kombination X, R¹, R² ist eine konstitutive Einheit der Formel (I), in der X für Sauerstoff steht, p null ist und R¹ für Wasserstoff steht.

Eine weitere besonders bevorzugte Substituenten-Kombination X, R¹, R² ist eine konstitutive Einheit der Formel (I), in der X für Sauerstoff steht, p null ist und R¹ für eine Acetylgruppe steht.

Weitere erfindungsgemäß bevorzugte Polymere A enthalten zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetyl-gruppe steht, wobei das Polymer A keine permanent-ionischen konstitutiven Einheiten enthält. Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten zu 65-25 Mol-% konstitutive Einheiten der Formel (Ia) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 65-25 Mol-% konstitutive Einheiten der Formel (Ia) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Das mindestens eine Polymer A mit mindestens zehn konstitutiven Einheiten der Formel (I) weist keine permanenten ionischen Ladungen auf. Es ist aber möglich, dass die konstitutiven Einheiten der Formel (I), beispielsweise durch Protonierung des Stickstoffatoms in einem sauren Träger, ionisch, insbesondere kationisch, vorliegen. Diese Ladungen sind aber nicht permanent, sondern temporär, da sie vom umgebenden Medium abhängig sind.

Bevorzugte erfindungsgemäße Färbe- oder Blondiermittel enthalten das mindestens eine Polymer A mit mindestens zehn konstitutiven Einheiten der Formel (I) in einer Gesamtmenge von 0,2 bis 5 Gew.-%, bevorzugt 0,5 bis 3 Gew.-%, besonders bevorzugt 1,0 bis 2,3 Gew.-%, jeweils bezogen auf das Gewicht des Färbe- oder Blondiermittels.

Als weiteren optionalen Inhaltsstoff enthalten erfindungsgemäß bevorzugte Färbe- oder Blondiermittel mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer ersten bevorzugten Ausführungsform enthalten erfindungsgemäß bevorzugte Färbe- oder Blondiermittel mindestens ein permanent kationisches Polymer, das ausgewählt ist aus
- kationischen Polymeren, die aufgebaut sind aus Monomeren mit quartären Ammoniumgruppen der allgemeinen Formel (IIa),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen,
   R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   a) kationischen Monomeren mit quartären Ammoniumgruppen der allgemeinen Formel (IIa),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen,
      R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   b) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die kationischen Monomere im molaren Überschuss zu den anionischen Monomeren vorliegen;
   außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind,
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
- sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweier- und Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweier- und Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße Färbe- oder Blondiermittel enthalten das mindestens eine permanent kationische Polymer B in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

Weitere erfindungsgemäß bevorzugte Färbe- oder Blondiermittel enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

Weitere erfindungsgemäß bevorzugte Färbe- oder Blondiermittel enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels. Weitere besonders bevorzugte Färbe- oder Blondiermittel enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

Weitere besonders bevorzugte Färbe- oder Blondiermittel enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39 in einer Gesamtmenge von 0,05 bis 1,5 Gew.-%, bevorzugt 0,1 bis 1,0 Gew.-%, besonders bevorzugt 0,2 bis 0,8 Gew.-%, jeweils bezogen auf das Gewicht des erfindungsgemäßen Färbe- oder Blondiermittels.

### Optionale Aminosäuren

Optional können die erfindungsgemäßen Färbe- oder Blondiermittel mindestens eine weitere Aminosäure enthalten, die von den Aminosäuren der Formel (VI) verschieden ist. Erfindungsgemäß bevorzugte optionale Aminosäuren sind ausgewählt aus Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren. Die optionalen Aminosäuren können auch in Salzform vorliegen, wobei hier die gleichen Salze bzw. Gegenionen bevorzugt sind wie für die vorstehend genannten gesättigten Dicarbonsäuren.

Überraschend hat sich herausgestellt, dass ein Gehalt an mindestens einer Aminosäure, die ausgewählt ist aus Serin, Alanin, Asparaginsäure, Glutaminsäure, Glycin, Isoleucin, Leucin, Phenylalanin, Prolin, Threonin, Tyrosin und Valin sowie Mischungen dieser Aminosäuren, die besonders geringe Haarschädigungswirkung der erfindungsgemäßen Färbe- oder Blondiermittel weiter reduzieren kann.

Eine besonders gute Wirkung konnte insbesondere für Serin festgestellt werden. Außerordentlich bevorzugte erfindungsgemäße Färbe- oder Blondiermittel enthalten Serin und mindestens eine der basischen Aminosäuren Arginin, Histidin oder Lysin, wobei besonders bevorzugt Serin und mindestens eine der basischen Aminosäuren Arginin, Histidin oder Lysin im Molverhältnis von Serin zu basischen Aminosäuren insgesamt im Bereich von 1:1 bis 50:1, bevorzugt 5:1 bis 30:1, enthalten sind.

Weitere erfindungsgemäß bevorzugte Färbe- oder Blondiermittel sind dadurch gekennzeichnet, dass die mindestens eine optionale Aminosäure in einer Gesamtmenge von 0,5 bis 5 Gew.-%, bevorzugt 0,7 bis 3 Gew.-%, besonders bevorzugt 0,9 bis 2 Gew.-%, jeweils bezogen auf das Gewicht des Färbe- oder Blondiermittels, enthalten ist.

Ein weiterer Gegenstand der vorliegenden Erfindung ist ein Verfahren zur oxidativen Färbung und/oder Aufhellung keratinischer Fasern, insbesondere von Humanhaar, bei dem ein Färbe- oder Blondiermittel auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird, wobei dieses Färbe- oder Blondiermittel
a) mindestens eine gesättigte Dicarbonsäure mit 2 bis 10 Kohlenstoffatomen und/oder mindestens ein Salz dieser Säure(n),
b) mindestens eine Aminosäure der Formel (VI) 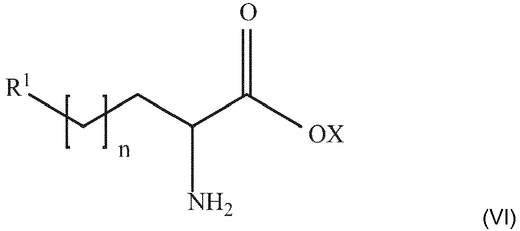 worin
   X für ein Wasserstoffatom oder ein ein- oder zweiwertiges Kation steht;
   n für null, 1, 2 oder 3 steht;
   R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung
enthält.

Für das erfindungsgemäße Verfahren zur oxidativen Färbung und/oder Aufhellung keratinischer Fasern, insbesondere von Humanhaar, und seine bevorzugten Ausführungsformen gilt mutatis mutandis das zu den erfindungsgemäßen und erfindungsgemäß bevorzugten Färbe- und Blondiermitteln Gesagte.

An das erfindungsgemäße Verfahren zur oxidativen Färbung und/oder Aufhellung keratinischer Fasern, insbesondere von Humanhaar, können sich optional weitere Haarbehandlungsschritte anschließen, beispielsweise die Applikation eines Conditioners, eines Haarverformungsmittels, beispielsweise eines Glättmittels oder eines Wellmittels, eines weiteren Haarfärbemittels, beispielsweise zum Färben oder Blondieren von Strähnchen, Ausspülschritte und Trocknungsschritte, zum Beispiel das Trockenreiben oder -pressen mit dem Handtuch, Fönen oder das Trocknen mit einer Trockenhaube.

Es kann erfindungsgemäß bevorzugt sein, die erfindungsgemäße Haar schützende Kombination aus mindestens einer gesättigten Dicarbonsäure mit 2 bis 10 Kohlenstoffatomen und/oder mindestens einem Salz dieser Säure(n) und mindestens einer Aminosäure der Formel (VI), wie vorstehend erläutert, zunächst getrennt von der Zubereitung, die das mindestens eine Alkalisierungsmittel und ggf. mindestens ein Oxidationsfarbstoffvorprodukt und/oder mindestens einen direktziehenden Farbstoff enthält, und getrennt von der Oxidationsmittelzubereitung, die mindestens eine Peroxidverbindung enthält, aufzubewahren und erst kurz vor Beginn des erfindungsgemäßen oder erfindungsgemäß bevorzugten Verfahrens zur oxidativen Färbung und/oder Aufhellung der keratinischen Fasern das erfindungsgemäße oder erfindungsgemäß bevorzugte Färbe- oder Blondiermittel durch Vermischen der drei Komponenten herzustellen.

Ein weiterer Gegenstand der vorliegenden Anmeldung ist daher ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (A), enthaltend
   a) mindestens eine gesättigte Dicarbonsäure mit 2 bis 10 Kohlenstoffatomen und/oder mindestens ein Salz dieser Säure(n),
   b) mindestens eine Aminosäure der Formel (VI) 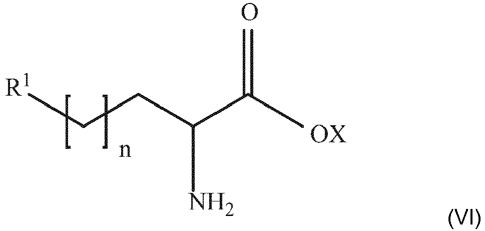 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe - SCH₃, oder mindestens ein Salz dieser Aminosäure,
   c) Wasser und
   d) optional weiterhin mindestens eine Substanz, die ausgewählt ist aus
      - Verbindungen der allgemeinen Formel (III), 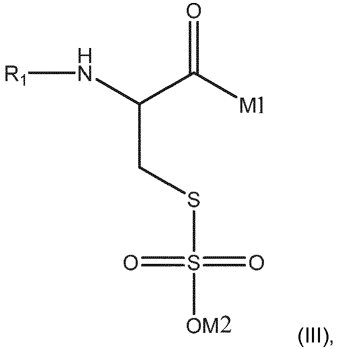 wobei
         R1 für ein Wasserstoffatom oder für ein Strukturelement der Formel (IV) steht 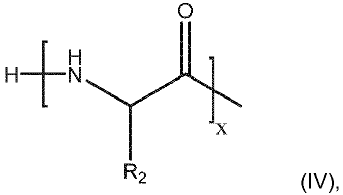 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
         M1 steht für die Gruppierung -OM2 oder für ein Strukturelement der Formel (V) 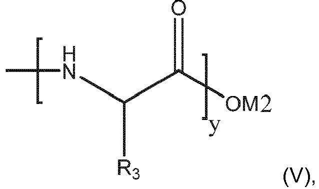 wobei
            y für eine ganze Zahl von 1 bis 100 steht,
            der Rest R3 in jedem der Strukturelemente der Formel (V) jeweils unabhängig vom vorangegangenen Strukturelement der Formel (V) gewählt werden kann,
            R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
            M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 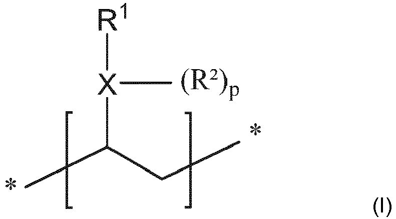 worin
         - X für Stickstoff oder Sauerstoff steht und
         - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 -C6-Alkylgruppe und/oder mit mindestens einer funktionellen Gruppe substituiert ist, und
         - p = 0 ist, wenn X für Sauerstoff steht und p = 1 ist, wenn X für Stickstoff steht,
      wobei das Polymer A keine permanent-ionischen konstitutiven Einheiten enthält,
II. Bereitstellen einer Zusammensetzung (B), enthaltend
   e) mindestens ein Alkalisierungsmittel, ausgewählt aus Ammoniumhydroxid, Monoethanolamin und Natriumsilikaten sowie Mischungen hiervon,
   f) ggf. Wasser und
   g) ggf. mindestens ein Oxidationsfarbstoffvorprodukt und/oder mindestens einen direktziehenden Farbstoff,
III. Bereitstellen einer Zusammensetzung (C), enthaltend
   h) mindestens eine Peroxidverbindung, die bevorzugt Wasserstoffperoxid ist,
IV. Vermischen der Zusammensetzungen (A), (B) und (C) miteinander, direkt anschließend
V. Auftragen der Mischung aus (A), (B) und (C) auf die keratinischen Fasern, insbesondere auf das Humanhaar, und
VI. Ausspülen nach einer Einwirkzeit von 0,1 bis 60 Minuten, bevorzugt 1 bis 45 Minuten, besonders bevorzugt 10 bis 30 Minuten,
VII. ggf. weitere Haarbehandlungen, wie Verformen, Konditionieren und/oder Trocknen.

### Mischungsverhältnisse von Zusammensetzung (A), Zusammensetzung (B) und Zusammensetzung (C)

Es hat sich erfindungsgemäß bewährt, wenn das Gewichtsverhältnis aus der Zusammensetzung (A), die Wasser und die Haar schützende Kombination aus mindestens einer gesättigten Dicarbonsäure mit 2 bis 10 Kohlenstoffatomen und mindestens einer Aminosäure der Formel (VI) enthält, der oxidativ färbenden und/oder oxidativ aufhellenden Mischung aus Zusammensetzung (B), enthaltend mindestens ein Alkalisierungsmittel und ggf. Oxidationsfarbstoffvorprodukte und/oder direktziehende Farbstoffe und Zusammensetzung (C), enthaltend mindestens eine Peroxidverbindung, im Bereich von [Gewicht von A]/[[Gewicht von B + Gewicht von C] wie 1:4 bis 1:50, bevorzugt 1:5 bis 1:25, besonders bevorzugt 1:9 bis 1:20, außerordentlich bevorzugt 1:10 bis 1:15.

Erfindungsgemäß bevorzugte Färbe- oder Aufhellverfahren mit den mindestens drei vorgenannten Zusammensetzungen (A), (B) und (C) sind dadurch gekennzeichnet, dass die mindestens eine gesättigte Dicarbonsäure mit 2 bis 10 Kohlenstoffatomen in der Zusammensetzung (A) in einer Gesamtmenge von 2 bis 20 Gew.-%, bevorzugt 5 bis 15 Gew.-%, besonders bevorzugt 8 bis 12 Gew.-%, enthalten ist, jeweils umgerechnet auf die undissoziierte Dicarbonsäure und bezogen auf das Gewicht der Zusammensetzung (A), enthalten ist.

Erfindungsgemäß bevorzugte Färbe- oder Aufhellverfahren mit den mindestens drei vorgenannten Zusammensetzungen (A), (B) und (C) sind weiterhin dadurch gekennzeichnet, dass die mindestens eine Aminosäure der Formel (VI) und/oder ein Salz hiervon in der Zusammensetzung (A) in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), enthalten ist.

Erfindungsgemäß bevorzugte Färbe- oder Aufhellverfahren mit den mindestens drei vorgenannten Zusammensetzungen (A), (B) und (C) sind weiterhin dadurch gekennzeichnet, dass mindestens ein Polymer A, das mindestens zehn konstitutive Einheiten der Formel (I) aufweist, 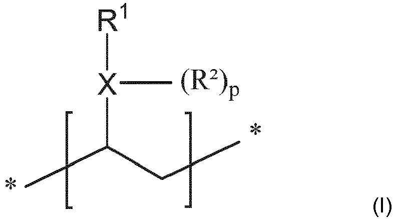 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält, in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A), enthalten ist.

Weitere erfindungsgemäß bevorzugte Färbe- oder Aufhellverfahren mit den mindestens drei vorgenannten Zusammensetzungen (A), (B) und (C) sind dadurch gekennzeichnet, dass in der Zusammensetzung (A), jeweils umgerechnet auf die undissoziierte Bernsteinsäure, 2 bis 20 Gew.-%, bevorzugt 5 bis 15 Gew.-%, besonders bevorzugt 8 bis 12 Gew.-%, Bernsteinsäure und mindestens einer der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, enthalten sind, jeweils umgerechnet auf die undissoziierte Aminosäure und bezogen auf das Gewicht der Zusammensetzung (A).

Weitere erfindungsgemäß bevorzugte Färbe- oder Aufhellverfahren mit den mindestens drei vorgenannten Zusammensetzungen (A), (B) und (C) sind dadurch gekennzeichnet, dass in Zusammensetzung (A) eine oder mehrere Verbindungen der vorstehend aufgeführten Formel (III) in einer Gesamtmenge von 0,001 bis 1 Gew.-% weiter bevorzugt von 0,005 bis 0,5 Gew.-% und besonders bevorzugt von 0,01 bis 0,1 Gew.-% enthalten ist, jeweils bezogen auf das Gewicht der Zusammensetzung (A).

Weitere erfindungsgemäß bevorzugte Färbe- oder Aufhellverfahren mit den mindestens drei vorgenannten Zusammensetzungen (A), (B) und (C) sind dadurch gekennzeichnet, dass in Zusammensetzung (A) 50 - 92 Gew.-%, bevorzugt 60 - 87 Gew.-% und besonders bevorzugt von 65 bis 80 Gew.-% Wasser enthalten ist, jeweils bezogen auf das Gewicht der Zusammensetzung (A). Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (A) einen pH-Wert im Bereich von 3,5 bis 7,1, bevorzugt 4,5 - 6,5, besonders bevorzugt 5,0 bis 6,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (B) einen pH-Wert im Bereich von 6,5 bis 11,0, bevorzugt 8 - 10,5, besonders bevorzugt 8,5 bis 10,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (C) einen pH-Wert im Bereich von 2,5 bis 6,5, bevorzugt 3,0 - 5,5, besonders bevorzugt 3,5 bis 5,0, aufweist, jeweils gemessen bei 20°C.

Weitere erfindungsgemäß bevorzugte Färbe- oder Aufhellverfahren mit den mindestens drei vorgenannten Zusammensetzungen (A), (B) und (C) sind dadurch gekennzeichnet, dass die Zusammensetzung (C) 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthält, jeweils bezogen auf das Gewicht der Zusammensetzung (C).

Für das erfindungsgemäße Verfahren zur oxidativen Färbung und/oder Aufhellung keratinischer Fasern, insbesondere von Humanhaar, mit dem aus den mindestens drei vorstehend genannten Zusammensetzungen (A), (B) und (C) und seine bevorzugten Ausführungsformen gilt - abgesehen von den geänderten quantitativen Angaben, die vorstehend genannt sind - mutatis mutandis das zu den erfindungsgemäßen und erfindungsgemäß bevorzugten Färbe- und Blondiermitteln Gesagte.

Ein weiterer Gegenstand der vorliegenden Erfindung ist eine Zusammensetzung (A) zur Haarbehandlung, enthaltend
- mindestens eine gesättigte Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure bevorzugt ausgewählt ist aus Bernsteinsäure, Äpfelsäure, Oxalsäure, Malonsäure, Adipinsäure, Pimelinsäure, Korksäure, Azelainsäure, Sebacinsäure, sowie Mischungen dieser Säuren, weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), und
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A).

Ein weiterer Gegenstand der vorliegenden Erfindung ist eine Zusammensetzung (A) zur Haarbehandlung, enthaltend
- mindestens eine gesättigte Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure bevorzugt ausgewählt ist aus Bernsteinsäure, Äpfelsäure, Oxalsäure, Malonsäure, Adipinsäure, Pimelinsäure, Korksäure, Azelainsäure, Sebacinsäure, sowie Mischungen dieser Säuren, weiterhin
- mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), und
- 50 - 92 Gew.-%, besonders bevorzugt 60 - 87 Gew.-% und außerordentlich bevorzugt von 65 bis 80 Gew.-% Wasser, jeweils bezogen auf das Gewicht der Zusammensetzung (A).

Ein weiterer Gegenstand der vorliegenden Erfindung ist eine Zusammensetzung (A) zur Haarbehandlung, enthaltend
- mindestens eine gesättigte Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure bevorzugt ausgewählt ist aus Bernsteinsäure, Äpfelsäure, Oxalsäure, Malonsäure, Adipinsäure, Pimelinsäure, Korksäure, Azelainsäure, Sebacinsäure, sowie Mischungen dieser Säuren, weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), weiterhin
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A), und
- mindestens ein Polymer A, das mindestens zehn konstitutive Einheiten der Formel (I) aufweist, 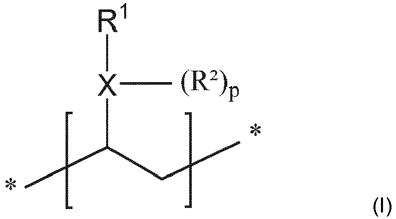 worin
   - X für Stickstoff oder Sauerstoff steht und
   - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
   - p = 0 ist, wenn X für Sauerstoff steht und p = 1 ist, wenn X für Stickstoff steht,
   wobei das Polymer A keine permanent-ionischen konstitutiven Einheiten enthält, in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

Ein weiterer Gegenstand der vorliegenden Erfindung ist eine Zusammensetzung (A) zur Haarbehandlung, enthaltend
- jeweils umgerechnet auf die undissoziierte Bernsteinsäure und bezogen auf das Gewicht der Zusammensetzung (A), 2 bis 20 Gew.-%, bevorzugt 5 bis 15 Gew.-%, besonders bevorzugt 8 bis 12 Gew.-%, Bernsteinsäure, weiterhin
- mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), weiterhin
- 50 - 92 Gew.-%, besonders bevorzugt 60 - 87 Gew.-% und außerordentlich bevorzugt von 65 bis 80 Gew.-% Wasser, jeweils bezogen auf das Gewicht der Zusammensetzung (A), und
- mindestens ein Polymer A, das mindestens zehn konstitutive Einheiten der Formel (I) aufweist, 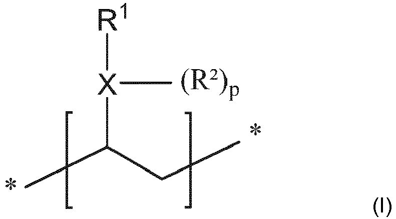 worin
   - X für Stickstoff oder Sauerstoff steht und
   - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
   - p = 0 ist, wenn X für Sauerstoff steht und p = 1 ist, wenn X für Stickstoff steht,
wobei das Polymer A keine permanent-ionischen konstitutiven Einheiten enthält,
in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

Ein weiterer Gegenstand der vorliegenden Erfindung ist eine Zusammensetzung (A) zur Haarbehandlung, enthaltend
- jeweils umgerechnet auf die undissoziierte Bernsteinsäure und bezogen auf das Gewicht der Zusammensetzung (A), 2 bis 20 Gew.-%, bevorzugt 5 bis 15 Gew.-%, besonders bevorzugt 8 bis 12 Gew.-%, Bernsteinsäure, weiterhin
- mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), weiterhin
- 50 - 92 Gew.-%, besonders bevorzugt 60 - 87 Gew.-% und außerordentlich bevorzugt von 65 bis 80 Gew.-% Wasser, jeweils bezogen auf das Gewicht der Zusammensetzung (A), und
- mindestens ein Polymer A, das mindestens zehn konstitutive Einheiten der Formel (I) aufweist, 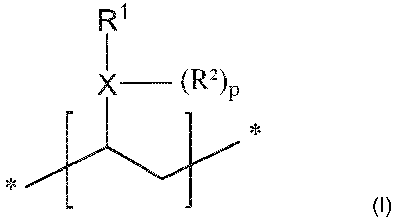 worin
   - X für Stickstoff oder Sauerstoff steht und
   - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
   - p = 0 ist, wenn X für Sauerstoff steht und p = 1 ist, wenn X für Stickstoff steht,
   wobei das Polymer A keine permanent-ionischen konstitutiven Einheiten enthält und ausgewählt ist aus Polyvinylpyrrolidon, Polyvinylalkohol sowie aus Mischungen hiervon, wobei Polyvinylpyrrolidon besonders bevorzugt ist,
   in der Zusammensetzung (A) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

Das in erfindungsgemäß bevorzugten Zusammensetzungen (A) enthaltene Polymer A weist mindestens zehn konstitutive Einheiten der Formel (I) auf, 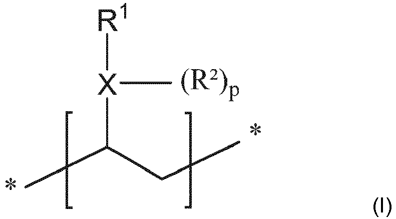 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält.

Überraschend wurde festgestellt, dass ein Polymer A, wie vorstehend bezeichnet und nachstehend näher erläutert, die Schutz- und Reparaturwirkung, die die Kombination aus mindestens einer gesättigten Dicarbonsäure mit 2 bis 10 Kohlenstoffatomen und/oder mindestens einem Salz dieser Säure(n) und mindestens einer Aminosäure der Formel (VI), wie vorstehend erläutert, auf oxidativ gefärbte oder blondierte keratinische Fasern ausübt, in hervorragender Weise unterstützt.

Unter "Polymer" werden im Sinne der vorliegenden Anmeldung Polymere im Sinne der IUPAC-Definition verstanden, die mindestens 10 identische konstitutive Einheiten umfassen.

Gemäß dem RÖMPP Chemie Lexikon, Stand Juli 2009, wird nach einer Definition der IUPAC eine Substanz als Polymer bezeichnet, die sich aus einem Kollektiv chemisch einheitlich aufgebauter Makromoleküle (Polymermoleküle) zusammensetzt, wobei sich diese Makromoleküle oder Polymermoleküle hinsichtlich Polymerisationsgrad, Molmasse und Kettenlänge voneinander unterscheiden. Bei derartigen so genannten polymereinheitlichen Stoffen sind also alle Makromoleküle gleich aufgebaut und unterscheiden sich lediglich durch ihre Kettenlänge (Polymerisationsgrad). Nach dieser IUPAC-Definition ist ein Polymer ferner "ein Polyreaktionsprodukt, das aus einer Vielzahl von Molekülen aufgebaut ist, in denen eine Art oder mehrere Arten von Atomen oder Atomgruppierungen (so genannte konstitutive Einheiten, Grundbausteine oder Wiederholungseinheiten) wiederholt aneinander gereiht sind.

Die Anzahl an konstitutiven Einheiten in einem Polymer bezeichnet man als Polymerisationsgrad. Erfindungsgemäß bevorzugte Polymere A wie auch Polymere B weisen jeweils einen Polymerisationsgrad im Bereich von 40 bis 1000, bevorzugt 100 bis 800, besonders bevorzugt 350 bis 650, auf. Weitere erfindungsgemäß bevorzugte Polymere A mit mindestens zehn konstitutiven Einheit der Formel (I) enthalten 40 bis 1000, bevorzugt 100 bis 800, besonders bevorzugt 350 bis 650 identische konstitutive Einheiten der Formel (I).

R¹ und R² stehen bevorzugt jeweils unabhängig voneinander für Wasserstoff oder für eine C₂-C₁₀-Acylgruppe, die bevorzugt ausgewählt aus einer Acetyl-, Propanoyl-, oder n-Butanoyl-Gruppe, besonders bevorzugt ausgewählt aus einer Acetyl-Gruppe.

Erfindungsgemäß bevorzugte Polymere A weisen mindestens 10 konstitutive Einheiten der Formel (I) auf, in denen X für Stickstoff steht, wobei das Polymer A keine permanent-ionischen konstitutiven Einheiten enthält.

Weitere erfindungsgemäß besonders bevorzugte Polymere A weisen mindestens 10 konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist.

Wenn R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, so ist dieser Ring bevorzugt mit mindestens einer funktionellen Gruppe substituiert, die ausgewählt ist aus =O. Ein besonders bevorzugte Substituenten-Kombination X, R¹, R² ist eine Pyrrolidon-Gruppe, so dass es sich bei einer erfindungsgemäß besonders bevorzugten konstitutiven Einheit der Formel (I) um eine Einheit der Formel (la) handelt, 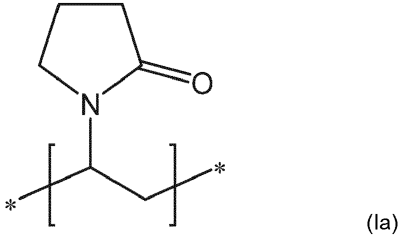 in der X für Stickstoff steht und R¹ und R² zusammen mit diesem Stickstoffatom einen fünfgliedrigen gesättigten Ring bilden, der keine weiteren Heteroatome enthält und der in 2-Position mit einer funktionellen Gruppe =O substituiert ist.

Eine weitere besonders bevorzugte Substituenten-Kombination X, R¹, R² ist eine ε-Caprolactam-Gruppe, so dass es sich bei einer erfindungsgemäß besonders bevorzugten konstitutiven Einheit der Formel (I) um eine Einheit der Formel (I b) handelt, 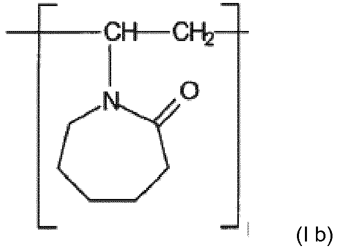 in der X für Stickstoff steht und R¹ und R² zusammen mit diesem Stickstoffatom einen sechsgliedrigen gesättigten Ring bilden, der keine weiteren Heteroatome enthält und der mit einer funktionellen Gruppe =O substituiert ist.

Eine weitere besonders bevorzugte Substituenten-Kombination X, R¹, R² ist eine Imidazol-Gruppe, so dass es sich bei einer weiteren erfindungsgemäß besonders bevorzugten Einheit der Formel (I) um eine Einheit handelt, in der X für Stickstoff steht und R¹ und R² zusammen mit diesem Stickstoffatom einen fünfgliedrigen ungesättigten Ring bilden, der als weiteres Heteroatom Stickstoff enthält. Weitere erfindungsgemäß bevorzugte Polymere A weisen zu 25 - 100 Mol-%, bevorzugt zu 55-100 Mol-%, besonders bevorzugt zu 85 - 100 Mol-% konstitutive Einheiten der Formel (I) auf, in denen X für Stickstoff steht, wobei das Polymer A keine permanent-ionischen konstitutiven Einheiten enthält.

Weitere erfindungsgemäß bevorzugte Polymere A weisen zu 25 - 100 Mol-%, bevorzugt zu 55-100 Mol-%, besonders bevorzugt zu 85 - 100 Mol-% konstitutive Einheiten der Formel (I) auf, in denen X für Stickstoff steht und R¹ und R² zusammen mit X einen fünf- oder sechsgliedrigen, gesättigten oder ungesättigten Ring bilden, der gegebenenfalls weitere Heteroatome enthält, die ausgewählt sind aus N und O und gegebenenfalls mit mindestens einer C1 - C6-Alkylgruppe und/oder mit mindestens einer funktionellen Gruppe substituiert ist, wobei das Polymer A keine permanent-ionischen konstitutiven Einheiten enthält.

Erfindungsgemäß besonders bevorzugte Polymere A weisen zu 98- 100 Mol-% konstitutive Einheiten der Formel (la) auf, wobei das Polymer A keine permanent-ionischen konstitutiven Einheiten enthält.

Erfindungsgemäß außerordentlich bevorzugte Polymere A weisen zu 98 - 100 Mol-% konstitutive Einheiten der Formel (la) auf und haben einen Polymerisationsgrad im Bereich von 40 bis 1000, bevorzugt 100 bis 800, besonders bevorzugt 350 bis 650, wobei das Polymer A keine permanent-ionischen konstitutiven Einheiten enthält. Besonders bevorzugte Polymere A sind Polyvinylpyrrolidon-Homopolymere mit einem Polymerisationsgrad im Bereich von 40 bis 1000, bevorzugt 100 bis 800, besonders bevorzugt 350 bis 650.

Eine weitere besonders bevorzugte Substituenten-Kombination X, R¹, R² ist eine konstitutive Einheit der Formel (I), in der X für Sauerstoff steht, p null ist und R¹ für Wasserstoff steht.

Eine weitere besonders bevorzugte Substituenten-Kombination X, R¹, R² ist eine konstitutive Einheit der Formel (I), in der X für Sauerstoff steht, p null ist und R¹ für eine Acetylgruppe steht.

Weitere erfindungsgemäß bevorzugte Polymere A enthalten zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetyl-gruppe steht, wobei das Polymer A keine permanent-ionischen konstitutiven Einheiten enthält. Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 75-92 Mol-% konstitutive Einheiten der Formel (I), in der X für Sauerstoff steht, p null ist und R¹ für Wasserstoff steht, und zu 8 - 25 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Weitere erfindungsgemäß bevorzugte Polymere A enthalten 40 bis 1000, bevorzugt 100 bis 800, besonders bevorzugt 350 bis 650 konstitutive Einheiten der Formel (I), davon zu 65-25 Mol-% konstitutive Einheiten der Formel (la) und zu 35 - 75 Mol-% konstitutive Einheiten der Formel (I), in der X für Sauerstoff steht, p null ist und R¹ für eine Acetylgruppe steht, wobei das Polymer A keine permanent-ionischen konstitutiven Einheiten enthält.

Das mindestens eine Polymer A mit mindestens zehn konstitutiven Einheiten der Formel (I) weist keine permanenten ionischen Ladungen auf. Es ist aber möglich, dass die konstitutiven Einheiten der Formel (I), beispielsweise durch Protonierung des Stickstoffatoms in einem sauren Träger, ionisch, insbesondere kationisch, vorliegen. Diese Ladungen sind aber nicht permanent, sondern temporär, da sie vom umgebenden Medium abhängig sind.

Bevorzugte erfindungsgemäße Zusammensetzungen (A) enthalten das mindestens eine Polymer A mit mindestens zehn konstitutiven Einheiten der Formel (I) in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A).

### Permanent kationisches Polymer B (optional)

Als weiteren optionalen Inhaltsstoff enthalten erfindungsgemäß bevorzugte Zusammensetzungen (A) mindestens ein permanent kationisches Polymer B.

Bevorzugt enthält das permanent kationische Polymer neben mindestens einem permanent kationisch geladenen Monomer-Typ auch mindestens einen permanent anionisch geladenen Monomer-Typ, wobei die kationischen Monomere im molaren Überschuss zu den anionischen Monomeren vorliegen, so dass das mindestens eine zweite erfindungsgemäße Polymer eine kationische Nettoladung aufweist. Derartige erfindungsgemäß bevorzugte Polymere werden auch als amphotere oder zwitterionische Polymere bezeichnet.

In einer ersten bevorzugten Ausführungsform enthalten erfindungsgemäß bevorzugte Zusammensetzungen (A) mindestens ein permanent kationisches Polymer, das ausgewählt ist aus
- kationischen Polymeren, die aufgebaut sind aus Monomeren mit quartären Ammoniumgruppen der allgemeinen Formel (IIa),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen,
   R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   c) kationischen Monomeren mit quartären Ammoniumgruppen der allgemeinen Formel (IIa),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   d) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die kationischen Monomere im molaren Überschuss zu den anionischen Monomeren vorliegen;
   außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind,
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
- sowie Mischungen der vorgenannten Polymere.

Erfindungsgemäß außerordentlich bevorzugte permanente kationische Polymere sind ausgewählt aus 2-[2-Hydroxy-3-(trimethylammonio)propoxy]ethylcelluloseetherchlorid, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen und Terpolymeren aus Acrylsäure, Diallyldimethylammoniumchlorid und Acrylamid, sowie Zweier- und Dreier-Mischungen dieser Polymere.

Besonders bevorzugte Polymer B-Mischungen enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid.

Erfindungsgemäß ebenfalls außerordentlich bevorzugte permanent-kationische Polymere B sind ausgewählt aus Polyquaternium-10, amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen, und Polyquaternium-39, sowie Zweier- und Dreier-Mischungen dieser Polymere.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht.

Weitere besonders bevorzugte Polymer B-Mischungen enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39.

Bevorzugte erfindungsgemäße Zusammensetzungen (A) enthalten das mindestens eine permanent kationische Polymer B in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere erfindungsgemäß bevorzugte Zusammensetzungen (A) enthalten mindestens ein permanent kationisches Polymer B, ausgewählt aus
- kationischen Polymeren, die aufgebaut sind aus Monomeren mit quartären Ammoniumgruppen der allgemeinen Formel (IIa),

   R³-CH=CR⁴-CO-Z-(CₙH₂ₙ)-N⁽⁺⁾R⁵R⁶R⁷ A⁽⁻⁾ (IIa),

   in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt,
   bevorzugt ausgewählt aus kationischen Polymeren, die aufgebaut sind aus Acrylamidopropyltrimethylammoniumchlorid,
   besonders bevorzugt ausgewählt aus amphoteren Polymeren mit kationischer Nettoladung, die durch Polymerisation aufgebaut sind aus
   e) kationischen Monomeren mit quartären Ammoniumgruppen der allgemeinen Formel (IIa),

      R³-CH=CR⁴-CO-Z-(CₙH₂ₙ)-N⁽⁺⁾R⁵R⁶R⁷ A⁽⁻⁾ (IIa),

      in der R³ und R⁴ unabhängig voneinander für Wasserstoff oder eine Methylgruppe stehen, R⁵, R⁶ und R⁷ unabhängig voneinander für eine Alkylgruppe mit 1 bis 4 Kohlenstoffatomen stehen, Z für eine NH-Gruppe oder ein Sauerstoffatom steht, n für eine ganze Zahl von 2 bis 4 steht und A⁽⁻⁾ das Anion einer anorganischen oder organischen Säure darstellt, und
   f) mindestens einer ungesättigten Carbonsäure, ausgewählt aus Acrylsäure, Methacrylsäure und Crotonsäure sowie aus Mischungen dieser Säuren, wobei die mindestens eine ungesättigte Carbonsäure in Form ihrer Salze vorliegen kann,
   wobei im Polymer die Gesamtheit aller kationischen Monomere im molaren Überschuss zur Gesamtheit aller anionischen Monomere vorliegt;
   außerordentlich bevorzugt ausgewählt aus amphoteren Polymeren mit kationischer Nettoladung, die den mindestens einen Monomer-Typ der allgemeinen Formel (IIa) und den mindestens einen Monomer-Typ der ungesättigten Carbonsäure, ausgewählt aus Acrylsäure, Methacrylsäure und Crotonsäure sowie Mischungen hiervon, in einem Molverhältnis von 60:40 bis 95:5, bevorzugt von 75:25 bis 90:10 zueinander enthalten,
   außerordentlichst bevorzugt ausgewählt aus amphoteren Copolymeren mit kationischer Nettoladung, die aus Acrylamidopropyltrimethylammoniumchlorid und Acrylsäure im Molverhältnis von 60:40 bis 95:5, bevorzugt von 75:25 bis 90:10 zueinander, bestehen;
- 2-[2-Hydroxy-3-(trimethylammonio)propoxy]ethylcelluloseetherchlorid, das beispielsweise unter der INCI-Bezeichnung Polyquaternium-10 erhältlich ist,
- Terpolymeren aus Acrylsäure, Diallyldimethylammoniumchlorid und Acrylamid, wie sie beispielsweise unter der INCI-Bezeichnung Polyquaternium-39 erhältlich sind,
- Homopolymeren des N,N,N-Trimethyl-2-[(methyl-1-oxo-2-propenyl)oxy]ethanaminiumchlorid, wie sie beispielsweise unter der INCI-Bezeichnung Polyquaternium-37 erhältlich sind,
- Copolymeren aus Diallyldimethylammoniumchlorid und Acrylsäure, wie sie beispielsweise unter der INCI-Bezeichnung Polyquaternium-22 erhältlich sind,
- Hydroxyethylcellulose-Dimethyldiallylammoniumchlorid-Copolymeren, wie sie beispielsweise unter der INCI-Bezeichnung Polyquaternium-4 erhältlich sind,
- Copolymeren aus Acrylamid und beta-Methacrylyloxyethyltrimethylammoniummethosulfat, wie sie beispielsweise unter der INCI-Bezeichnung Polyquaternium-5 erhältlich sind,
- Homopolymeren des N,N-Dimethyl-N-2-Propenyl-2-Propen-1-aminiumchlorid, wie sie beispielsweise unter der INCI-Bezeichnung Polyquaternium-6 erhältlich sind,
- Copolymeren aus Diallyldimethylammoniumchlorid und Acrylamid, wie sie beispielsweise unter der INCI-Bezeichnung Polyquaternium-7 erhältlich sind, sowie
- Copolymeren aus Vinylpyrrolidon und Dimethylaminoethylmethacrylatdiethylsulfat, wie sie beispielsweise unter der INCI-Bezeichnung Polyquaternium-11 erhältlich sind,
in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A). Weitere erfindungsgemäß bevorzugte Zusammensetzungen (A) enthalten 2-[2-Hydroxy-3-(trimethylammonio)propoxy]ethylcelluloseetherchlorid und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere erfindungsgemäß bevorzugte Zusammensetzungen (A) enthalten 2-[2-Hydroxy-3-(trimethylammonio)propoxy]ethylcelluloseetherchlorid, mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und mindestens ein Terpolymer aus Acrylsäure, Diallyldimethylammoniumchlorid und Acrylamid, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere besonders bevorzugte Zusammensetzungen (A) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Weitere besonders bevorzugte Zusammensetzungen (A) enthalten Polyquaternium-10 und mindestens ein amphoteres Copolymer mit kationischer Nettoladung, das aus Acrylamidopropyltrimethylammoniumchlorid und Acrylsäure im Molverhältnis von 60:40 bis 95:5, bevorzugt von 75:25 bis 90:10 zueinander, besteht, und Polyquaternium-39 in einer Gesamtmenge von 0,1 bis 5 Gew.-%, bevorzugt 0,2 bis 3 Gew.-%, besonders bevorzugt 0,5 bis 1,5 Gew.-%, jeweils bezogen auf das Gewicht der erfindungsgemäßen Zusammensetzung (A).

Optional kann die vorgenannte Zusammensetzung (A) weiterhin mindestens eine Substanz enthalten, die ausgewählt ist aus Verbindungen der allgemeinen Formel (III), 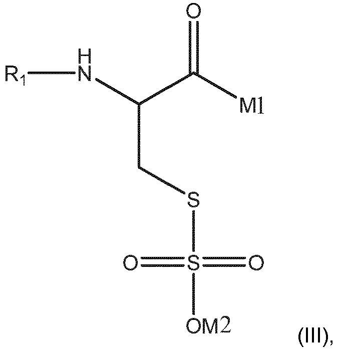 wobei
R1 für ein Wasserstoffatom oder für ein Strukturelement der Formel (IV) steht 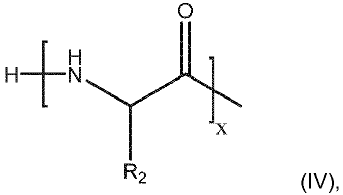 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)methyl-Gruppe,
M1 steht für die Gruppierung -OM2 oder für ein Strukturelement der Formel (V) 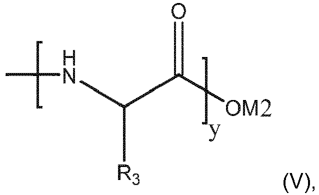 wobei
   y für eine ganze Zahl von 1 bis 100 steht,
   der Rest R3 in jedem der Strukturelemente der Formel (V) jeweils unabhängig vom vorangegangenen Strukturelement der Formel (V) gewählt werden kann,
   R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
   M2 steht für ein Wasserstoffatom, ein Äquivalent eines ein oder mehrwertigen Kations oder ein Ammoniumion (NH₄)⁺.

In einer ganz besonders bevorzugten Ausführungsform ist eine erfindungsgemäße Zusammensetzung (A) dadurch gekennzeichnet, dass sie mindestens eine Verbindung der Formel (III) enthält, die ein Molekulargewicht M_{w} von 200 bis 2.000 Da (Dalton), vorzugsweise von 250 bis 1.500 Da, bevorzugt von 300 bis 1.200 Da, insbesondere von 400 bis 800 Da aufweist.

Wird in der erfindungsgemäßen Zusammensetzung (A) eine Mischung von Oligomeren eingesetzt, so können diese Mischungen durch ihr mittleres Molekulargewicht definiert werden.

In diesem Fall ist eine erfindungsgemäß bevorzugte Zusammensetzung (A) dadurch gekennzeichnet, dass sie mindestens eine Mischung von Verbindungen der Formel (III) enthält, die ein mittleres Molekulargewicht M_{w} von 200 bis 2.000 Da, vorzugsweise von 250 bis 1.500 Da, bevorzugt von 300 bis 1.200 Da, insbesondere von 400 bis 800 Da aufweist.

Weiterhin hat sich gezeigt, dass der Schutz- oder Repair-Effekt, den die Verbindungen der Formel (III) besitzen, auch von den Wiederholungsindices x und y abhängt. Wie zuvor beschrieben, ist es ganz besonders bevorzugt, wenn x für eine ganze Zahl von 1 bis 10 steht und y für eine ganze Zahl von 1 bis 10 steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
M1 für ein Strukturelement der Formel (V) steht, und
x für eine ganze Zahl von 1 bis 10 steht und
y für eine ganze Zahl von 1 bis 10 steht.

Neben dem Molekulargewicht der Verbindung der Formel (III) nimmt auch der Anteil der Bunte-Salz Einheiten, die in der Verbindung der Formel (III) enthalten sind, einen entscheidenden Einfluss auf die Wirksamkeit der Schutzwirkung oder "Repair-Wirkung" der Verbindungen.

Verbindungen mit mindestens einer Bunte-Salz-Einheit - wie sie beispielsweise in der Verbindung der Formel (IIIa) vorhanden ist-sind sehr effektiv, insbesondere wenn sie als monomere Verbindung eingesetzt werden. Oligopeptide mit mindestens einer Bunte-Salz Einheit sind besonders wirksam, wenn sie ein niedriges Molekulargewicht von bis zu 1200, insbesondere 800 Dalton besitzen.

Bei Einsatz von Oligopeptiden ist es jedoch von ganz besonderem Vorteil, wenn die Verbindung der Formel (III) mindestens zwei, bevorzugt mindestens drei Bunte-Salz Einheiten besitzt.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
der Rest R2 in mindestens einem Strukturelement der Formel (IV) für eine
(Sulfosulfanyl)methyl-Gruppe (d.h. eine Gruppe HO-S(O₂)-S-CH₂-) steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
R1 für ein Strukturelement der Formel (IV) steht, und
x für eine ganze Zahl von mindestens 3 steht und
der Rest R2 in mindestens 3 Strukturelementen der Formel (IV) für eine 2-Carboxyethyl-Gruppe (d.h. eine Gruppe HOOC-CH2-CH2-) steht.

In einer weiteren ganz besonders bevorzugten Ausführungsform ist eine erfindungsgemäße Zusammensetzung (A) dadurch gekennzeichnet, dass sie mindestens eine Verbindung der Formel (III) enthält, wobei
M1 für ein Strukturelement der Formel (V) steht, und
y für eine ganze Zahl von mindestens 3 steht und
der Rest R3 in mindestens 3 Strukturelementen der Formel (IV) für eine Gruppe (Glu) steht. Die mindestens eine Verbindung der Formel (III) ist - bezogen auf das Gesamtgewicht der erfindungsgemäß bevorzugten Zusammensetzung (A) in einer Gesamtmenge von 0,001 bis 10 Gew.-% enthalten. Überraschenderweise hat sich jedoch herausgestellt, dass die Verbindung(en) der Formel (III) bereits in geringen Einsatzkonzentrationen eine sehr gute Reduzierung der Haarschädigung bewirken können. Dies ist insbesondere deshalb von Vorteil, weil die mindestens eine Verbindung der Formel (III) in der erfindungsgemäßen Zusammensetzung (A) als Additiv (beispielsweise in Form einer Pflegelösung oder Reparaturlösung) vor der Applikation auf das Haar hinzugefügt werden soll. Aus diesem Grund ist es besonders vorteilhaft, wenn die erfindungsgemäß bevorzugte Zusammensetzung (A) eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht der erfindungsgemäßen Zusammensetzung (A).

In einer weiteren ganz besonders bevorzugten Ausführungsform ist eine erfindungsgemäße Zusammensetzung (A) dadurch gekennzeichnet, dass sie eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthält, jeweils bezogen auf das Gewicht der erfindungsgemäßen Zusammensetzung (A), wobei in Formel (III) R1 für ein Strukturelement der Formel (IV) steht, und der Rest R2 in mindestens einem Strukturelement der Formel (IV) für eine (Sulfosulfanyl)methyl-Gruppe (d.h. eine Gruppe HO-S(O₂)-S-CH₂-) steht.

Weiterhin kann es erfindungsgemäß bevorzugt sein, die Haar schützende erfindungsgemäße Kombination aus mindestens einer gesättigten Dicarbonsäure mit 2 bis 10 Kohlenstoffatomen und mindestens einer Aminosäure der Formel (VI) zunächst zusammen mit der Zubereitung, die das mindestens eine Alkalisierungsmittel und ggf. mindestens ein Oxidationsfarbstoffvorprodukt und/oder mindestens einen direktziehenden Farbstoff enthält, aber getrennt von der Oxidationsmittelzubereitung, die mindestens eine Peroxidverbindung enthält, aufzubewahren und erst kurz vor Beginn des erfindungsgemäßen oder erfindungsgemäß bevorzugten Verfahrens zur oxidativen Färbung und/oder Aufhellung der keratinischen Fasern das erfindungsgemäße oder erfindungsgemäß bevorzugte Färbe- oder Blondiermittel durch Vermischen der beiden Komponenten herzustellen.

In einer weiteren ganz besonders bevorzugten Ausführungsform weist die erfindungsgemäße Zusammensetzung (A) einen pH-Wert im Bereich von 3,5 bis 7,1, bevorzugt 4,5 - 6,5, besonders bevorzugt 5,0 bis 6,0, auf, jeweils gemessen bei 20°C.

Ein weiterer Gegenstand der vorliegenden Anmeldung ist daher ein Verfahren zur oxidativen Färbung und/oder Aufhellung keratinischer Fasern, insbesondere von Humanhaar, das folgende Verfahrensschritte umfasst:
I. Bereitstellen einer Zusammensetzung (AB), enthaltend
   a) mindestens eine gesättigte Dicarbonsäure mit 2 bis 10 Kohlenstoffatomen und/oder mindestens ein Salz dieser Säure(n),
   b) mindestens eine Aminosäure der Formel (VI), 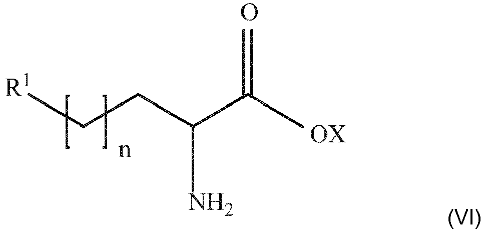 worin
      X für ein Wasserstoffatom oder ein ein- oder zweiwertiges Kation steht;
      n für null, 1, 2 oder 3 steht;
      R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃ oder mindestens ein Salz dieser Aminosäure,
      wobei die Aminosäure der Formel (VI) bevorzugt ausgewählt ist aus Arginin, Lysin, Histidin sowie Mischungen hiervon, besonders bevorzugt Mischungen aus Arginin und Lysin, oder mindestens einem Salz dieser Aminosäuren,
   c) optional weiterhin mindestens eine Substanz, die ausgewählt ist aus
      - Verbindungen der allgemeinen Formel (III), 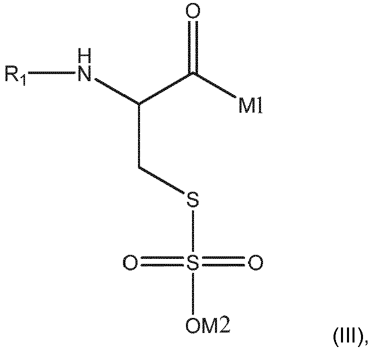 wobei
         R1 für ein Wasserstoffatom oder für ein Strukturelement der Formel (IV) steht 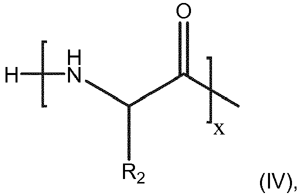 wobei
         x für eine ganze Zahl von 1 bis 100 steht,
         der Rest R2 in jedem der Strukturelemente der Formel (IV) jeweils unabhängig vom vorangegangenen Strukturelement der Formel (IV) gewählt werden kann,
         R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-lndol-3-ylmethyl-Gruppe oder eine (Sulfosulfanyl)-methyl-Gruppe,
         M1 steht für die Gruppierung -OM2 oder für ein Strukturelement der Formel (V) 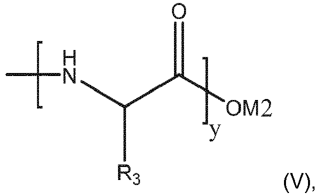 wobei
            y für eine ganze Zahl von 1 bis 100 steht,
            der Rest R3 in jedem der Strukturelemente der Formel (V) jeweils unabhängig vom vorangegangenen Strukturelement der Formel (V) gewählt werden kann,
            R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-lndol-3-ylmethyl-Gruppe oder eine (Sulfosulfanyl)-methyl-Gruppe,
            M2 steht für ein Wasserstoffatom, ein Äquivalent eines ein oder mehrwertigen Kations oder ein Ammoniumion (NH₄)⁺, und
      - Polymeren A, die mindestens zehn konstitutive Einheiten der Formel (I) aufweisen, 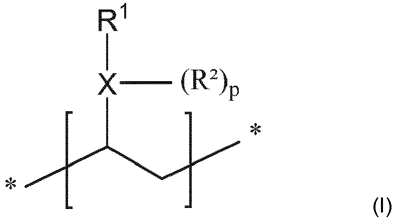 worin
         - X für Stickstoff oder Sauerstoff steht und
         - R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 -C6-Alkylgruppe und/oder mit mindestens einer funktionellen Gruppe substituiert ist, und
         - p = 0 ist, wenn X für Sauerstoff steht und p = 1 ist, wenn X für Stickstoff steht,
         wobei das Polymer A keine permanent-ionischen konstitutiven Einheiten enthält,
   d) mindestens ein Alkalisierungsmittel, ausgewählt aus Ammoniumhydroxid, Monoethanolamin und Natriumsilikaten sowie Mischungen hiervon,
   e) Wasser und
   f) ggf. mindestens ein Oxidationsfarbstoffvorprodukt und/oder mindestens einen direktziehenden Farbstoff,
II. Bereitstellen einer Zusammensetzung (C), enthaltend
   g) mindestens eine Peroxidverbindung, die bevorzugt Wasserstoffperoxid ist,
III. Vermischen der Zusammensetzungen (AB) und (C) miteinander, direkt anschließend
IV. Auftragen der Mischung aus (AB) und (C) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

### Mischungsverhältnisse der Zusammensetzung (AB) mit Zusammensetzung (C)

Es hat sich erfindungsgemäß bewährt, wenn das Gewichtsverhältnis aus der Zusammensetzung (AB), die die Haar schützende Kombination aus mindestens einem Polymer, das mindestens zehn konstitutive Einheiten der Formel (I) aufweist, weiterhin mindestens einem permanent kationischen Polymer, mindestens einer Aminosäure und Wasser in der alkalisierenden Zusammensetzung (B), enthaltend mindestens ein Alkalisierungsmittel und ggf. Oxidationsfarbstoffvorprodukte und/oder direktziehende Farbstoffe enthält und der Zusammensetzung (C), enthaltend mindestens eine Peroxidverbindung, im Bereich von [Gewicht von AB]/[[Gewicht von C] wie 1:0,8 bis 1:2,5, bevorzugt 1:1 bis 1:2 liegt.

Erfindungsgemäß bevorzugte Färbe- oder Aufhellverfahren mit den mindestens zwei vorgenannten Zusammensetzungen (AB) und (C) sind dadurch gekennzeichnet, dass die mindestens eine Dicarbonsäure mit 2 bis 10 Kohlenstoffatomen, die bevorzugt ausgewählt ist aus Bernsteinsäure, Äpfelsäure, Oxalsäure, Malonsäure, Adipinsäure, Pimelinsäure, Korksäure, Azelainsäure, Sebacinsäure, sowie Mischungen dieser Säuren, in der Zusammensetzung (AB) in einer auf die Masse an freier Dicarbonsäure umgerechneten Gesamtmenge von 0,03 - 7 Gew.-%, bevorzugt 0,1 - 5 Gew.-%, besonders bevorzugt 0,5 - 3 Gew.-%, außerordentlich bevorzugt 0,9 - 1,5 Gew.-%, jeweils bezogen auf das Gewicht der Zusammensetzung (AB), enthalten ist.

Erfindungsgemäß bevorzugte Färbe- oder Aufhellverfahren mit den mindestens zwei vorgenannten Zusammensetzungen (AB) und (C) sind dadurch gekennzeichnet, dass das mindestens eine Polymer A mit mindestens zehn konstitutiven Einheiten der Formel (I) in der Zusammensetzung (AB) in einer Gesamtmenge von 0,8 bis 10 Gew.-%, bevorzugt 2 bis 6 Gew.-%, besonders bevorzugt 3 bis 5 Gew.-%,jeweils bezogen auf das Gewicht der Zusammensetzung (AB), enthalten ist. Erfindungsgemäß bevorzugte Färbe- oder Aufhellverfahren mit den mindestens zwei vorgenannten Zusammensetzungen (AB) und (C) sind weiterhin dadurch gekennzeichnet, dass das mindestens eine permanent kationische Polymer B in der Zusammensetzung (AB) in einer Gesamtmenge von 0,1 bis 5 Gew.-%, bevorzugt 0,2 bis 3,0 Gew.-%, besonders bevorzugt 0,4 bis 1,5 Gew.-%, jeweils bezogen auf das Gewicht der Zusammensetzung (AB), enthalten ist.

Erfindungsgemäß bevorzugte Färbe- oder Aufhellverfahren mit den mindestens zwei vorgenannten Zusammensetzungen (AB) und (C) sind weiterhin dadurch gekennzeichnet, dass die mindestens eine Aminosäure der Formel (VI) in der Zusammensetzung (AB) in einer Gesamtmenge von 0,5 bis 8 Gew.-%, bevorzugt 0,9 bis 5 Gew.-%, besonders bevorzugt 2 bis 3 Gew.-%, jeweils umgerechnet auf die Masse an freier Aminosäure und bezogen auf das Gewicht der Zusammensetzung (AB), enthalten ist.

Weitere erfindungsgemäß bevorzugte Färbe- oder Aufhellverfahren mit den mindestens zwei vorgenannten Zusammensetzungen (AB) und (C) sind dadurch gekennzeichnet, dass eine Mischung aus Serin und mindestens einer der basischen Aminosäuren Arginin, Histidin oder Lysin in der Zusammensetzung (AB) in einer Gesamtmenge von 0,5 bis 8 Gew.-%, bevorzugt 0,9 bis 5 Gew.-%, besonders bevorzugt 2 bis 3 Gew.-%, jeweils umgerechnet auf die Masse an freier Aminosäure und bezogen auf das Gewicht der Zusammensetzung (AB), enthalten ist, wobei besonders bevorzugt Serin und mindestens eine der basischen Aminosäuren Arginin, Histidin oder Lysin im Molverhältnis von Serin zu basischen Aminosäuren insgesamt im Bereich von 1:1 bis 50:1, bevorzugt 5:1 bis 30:1, enthalten ist.

Weitere erfindungsgemäß bevorzugte Färbe- oder Aufhellverfahren mit den mindestens zwei vorgenannten Zusammensetzungen (AB) und (C) sind dadurch gekennzeichnet, dass in der Zusammensetzung (AB) eine oder mehrere Verbindungen der vorstehend aufgeführten Formel (III) in einer Gesamtmenge von 0,002 bis 2,5 Gew.-% weiter bevorzugt von 0,02 bis 2,0 Gew.-% und besonders bevorzugt von 0,04 bis 0,2 Gew.-% enthalten sind, jeweils bezogen auf das Gewicht der Zusammensetzung (AB), enthalten sind.

Weitere erfindungsgemäß bevorzugte Färbe- oder Aufhellverfahren mit den mindestens zwei vorgenannten Zusammensetzungen (AB) und (C) sind dadurch gekennzeichnet, dass die Zusammensetzung (C) 1,0 bis 23,0 Gew.-%, weiter bevorzugt 2,5 bis 21,0 Gew.-%, besonders bevorzugt 4,0 bis 20,0 Gew.-% und ganz besonders bevorzugt 5,0 bis 18,0 Gew.-% Wasserstoffperoxid (berechnet als 100 %-iges H₂O₂) enthält, jeweils bezogen auf das Gewicht der Zusammensetzung (C). Die erfindungsgemäßen Zusammensetzungen (AB) enthalten Wasser, und zwar bevorzugt in einer Menge von 20 bis 85 Gew.-%, bevorzugt 30 bis 80 Gew.-%, jeweils bezogen auf das Gesamtgewicht der erfindungsgemäßen Zusammensetzung (AB).

Die erfindungsgemäßen Zusammensetzungen (AB) weisen bevorzugt einen pH-Wert im Bereich von 6,5 bis 10,5, bevorzugt 8 - 10, besonders bevorzugt 8,5 bis 9,5, aufweist, jeweils gemessen bei 20°C.

Für das erfindungsgemäße Verfahren zur oxidativen Färbung und/oder Aufhellung keratinischer Fasern, insbesondere von Humanhaar, mit dem aus den mindestens zwei vorstehend genannten Zusammensetzungen (AB) und (C) und seine bevorzugten Ausführungsformen gilt - abgesehen von den geänderten quantitativen Angaben, die vorstehend genannt sind - mutatis mutandis das zu den erfindungsgemäßen und erfindungsgemäß bevorzugten Färbe- und Blondiermitteln Gesagte.

## Patentansprüche

1. Oxidatives Färbe- oder Blondiermittel für keratinische Fasern, insbesondere für Humanhaar, enthaltend
a) mindestens eine gesättigte Dicarbonsäure mit 2 bis 10 Kohlenstoffatomen und/oder mindestens ein Salz dieser Säure(n),
b) mindestens eine Aminosäure der Formel (VI) 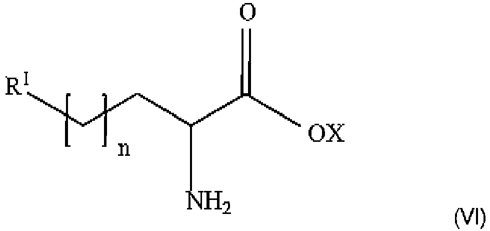 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-lmidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃. oder mindestens ein Salz dieser Aminosäure,
c) weiterhin mindestens ein Alkalisierungsmittel, ausgewählt aus Ammoniumhydroxid, Monoethanolamin und Natriumsilikaten sowie Mischungen hiervon,
d) ggf. mindestens ein Oxidationsfarbstoffvorprodukt und/oder mindestens einen direktziehenden Farbstoff,
e) Wasser und
f) mindestens eine Peroxidverbindung.

2. Färbe- oder Blondiermittel gemäß Anspruch 1, **dadurch gekennzeichnet, dass** die mindestens eine gesättigte Dicarbonsäure mit 2 bis 10 Kohlenstoffatomen ausgewählt ist aus Bernsteinsäure, Oxalsäure, Malonsäure, Adipinsäure, Pimelinsäure, Korksäure, Azelainsäure, Sebacinsäure, sowie Mischungen dieser Säuren, bevorzugt ausgewählt aus Bernsteinsäure.

3. Färbe- oder Blondiermittel gemäß Anspruch 1 oder 2, **dadurch gekennzeichnet, dass** die mindestens eine gesättigte Dicarbonsäure mit 2 bis 10 Kohlenstoffatomen in einer Gesamtmenge von 0,2 bis 4 Gew.-%, bevorzugt 0,33 bis 3 Gew.-%, besonders bevorzugt 0,5 bis 2 Gew.-%, enthalten ist, jeweils umgerechnet auf die undissoziierte Dicarbonsäure und bezogen auf das Gewicht des Färbe- oder Blondiermittels.

4. Färbe- oder Blondiermittel gemäß einem der Ansprüche 1 bis 3, **dadurch gekennzeichnet, dass** die mindestens eine Aminosäure der Formel (VI) ausgewählt ist aus Arginin, Lysin, Histidin, Asparagin, Glutamin, Cystein, Methionin, Tryptophan sowie Mischungen hiervon, besonders bevorzugt Mischungen aus Arginin und Lysin.

5. Färbe- oder Blondiermittel gemäß einem der Ansprüche 1 bis 4, **dadurch gekennzeichnet, dass** die mindestens eine Aminosäure der Formel (VI) in einer Gesamtmenge von 0,05 bis 3 Gew.-%, bevorzugt 0,1 bis 2 Gew.-%, besonders bevorzugt 0,2 bis 1,2 Gew.-%, enthalten ist, jeweils umgerechnet auf die undissoziierte Aminosäure und bezogen auf das Gewicht des Färbe- oder Blondiermittels.

6. Färbe- oder Blondiermittel gemäß einem der Ansprüche 1 - 5, **dadurch gekennzeichnet, dass** mindestens eine Verbindung der allgemeinen Formel (III) enthalten ist, 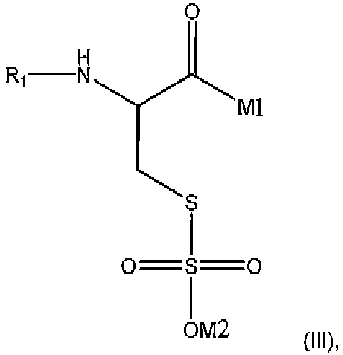 wobei
R1 für ein Wasserstoffatom oder für ein Strukturelement der Formel (IV) steht 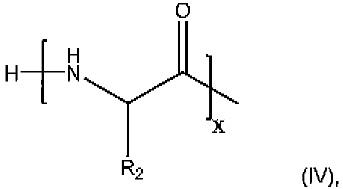 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, 1-Hydroxyethylgruppe, eine 4-AminobutyIgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyi)methyl-Gruppe,
M1 steht für die Gruppierung -OM2 oder für ein Strukturelement der Formel (V) 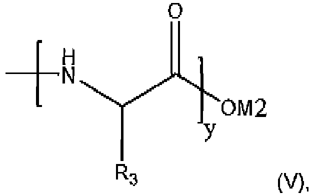 wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methyl-propylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺,
wobei bevorzugt eine oder mehrere Verbindungen der vorstehend aufgeführten Formel (III) in einer Gesamtmenge von 0,001 bis 2,5 Gew.-% weiter bevorzugt von 0,01 bis 1,0 Gew.-% und besonders bevorzugt von 0,02 bis 0,1 Gew.-% enthalten sind, jeweils bezogen auf das Gewicht des erfindungsgemaßen Färbe- oder Blondiermittels.

7. Färbe- oder Blondiermittel gemäß einem der Ansprüche 1 - 6, **dadurch gekennzeichnet, dass** als Peroxidverbindung Wasserstoffperoxid enthalten ist.

8. Farbe- oder Blondiermittel gemäß einem der Ansprüche 1 - 7, **dadurch gekennzeichnet, dass** mindestens ein Polymer A enthalten ist, das mindestens zehn konstitutive Einheiten der Formel (1) aufweist, 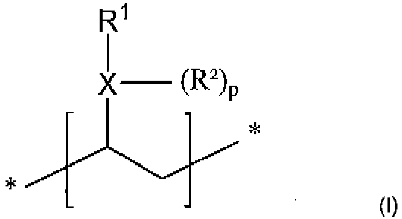 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält,
wobei das mindestens eine Polymer A mit mindestens zehn konstitutiven Einheiten der Formel (I) bevorzugt in einer Gesamtmenge von 0,2 bis 5 Gew.-%, besonders bevorzugt 0,5 bis 3 Gew.-%, außerordentlich bevorzugt 1,0 bis 2,3 Gew.-%, jeweils bezogen auf das Gewicht des Färbe- oder Blondiermittels, enthalten ist.

9. Färbe- oder Blondiermittel gemäß Anspruch 8, **dadurch gekennzeichnet, dass** das mindestens eine Polymer A mit mindestens zehn konstitutiven Einheiten der Formel (I) ausgewählt ist aus Polymeren, die zu 98 - 100 Mol-% konstitutive Einheiten der Formel (Ia) 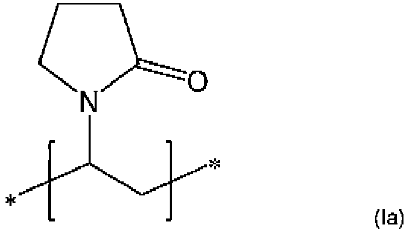 aufweisen und einen Polymerisationsgrad im Bereich von 40 bis 1000, bevorzugt 100 bis 800, besonders bevorzugt 350 bis 650 haben, wobei das Polymer A keine permanent-ionischen konstitutiven Einheiten enthält.

10. Verfahren zur oxidativen Färbung und/oder Aufhellung keratinischer Fasern, insbesondere von Humanhaar, bei dem ein Färbe- oder Blondiermittel gemäß einem der Ansprüche 1 - 9 auf die keratinischen Fasern, insbesondere auf das Humanhaar, aufgetragen und nach einer Einwirkzeit von 0,1 bis 60 Minuten, bevorzugt 1 bis 45 Minuten, besonders bevorzugt 10 bis 30 Minuten, wieder ausgespült wird.

11. Verfahren zur oxidativen Färbung und/oder Aufhellung keratinischer Fasern, insbesondere von Humanhaar, das folgende Verfahrensschritte umfasst:
I. Bereitstellen einer Zusammensetzung (A), enthaltend
a) mindestens eine gesättigte Dicarbonsäure mit 2 bis 10 Kohlenstoffatomen und/oder mindestens ein Salz dieser Säure(n),
b) mindestens eine Aminosäure der Formel (VI) 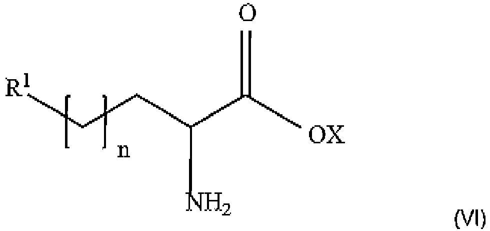 worin
X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsaureamid-Gruppe -CONH₂, einer 1*H*-Indol-3-yl-Gruppe einer Thiol-Gruppe -SH und einer Methylsulfanyl-Gruppe-SCH₃, oder mindestens ein Salz dieser Aminosäure,
c) Wasser und
d) optional weiterhin mindestens eine Substanz, die ausgewählt ist aus
- Verbindungen der allgemeinen Formel (III), 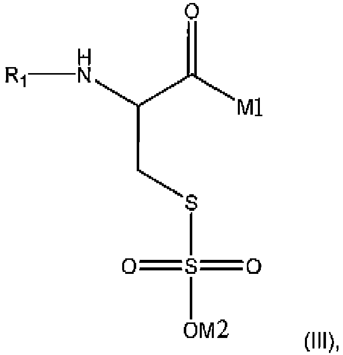 wobei
R1 für ein Wasserstoffatom oder für ein Strukturelement der Formel (IV) steht 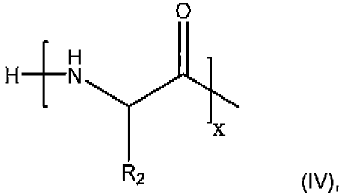 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 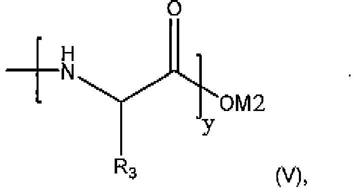 wobei
y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1 H-lndol-3-ylmethyl-Gruppe oder eine (Sulfosulfanyl)methy l-Gruppe,
M2 steht für ein Wasserstoffatom, ein Äquivalent eines ein oder mehrwertigen Kations oder ein Ammoniumion (NH₄)⁺, und
- Polymeren A, die mindestens zehn konstitutive Einheiten der Formel (I) aufweisen, 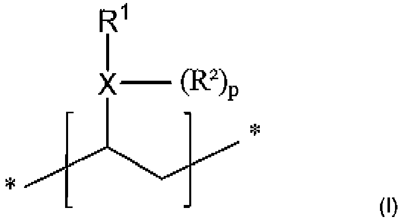 worin
- X für Stickstoff oder Sauerstoff steht und
- R¹ und R² jeweils unabhängig voneinander für Wasserstoff oder eine C2-C10-Acyl-gruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält, wobei die Zusammensetzung (A) bevorzugt einen pH-Wert im Bereich von 3,5 bis 7,1, bevorzugt 4,5 - 6,5, besonders bevorzugt 5,0 bis 6,0, aufweist, jeweils gemessen bei 20°C,
II. Bereitstellen einer Zusammensetzung (B), enthaltend
e) mindestens ein Alkalisierungsmittel, ausgewählt aus Ammoniumhydroxid, Monoethanolamin und Natriumsilikaten sowie Mischungen hiervon,
f) ggf. Wasser und
g) ggf. mindestens ein Oxidationsfarbstoffvorprodukt und/oder mindestens einen direktziehenden Farbstoff,
wobei die Zusammensetzung (B) bevorzugt einen pH-Wert im Bereich von 6,5 bis 11,0, bevorzugt 8 - 10,5, besonders bevorzugt 8,5 bis 10,0, aufweist, jeweils gemessen bei 20°C,
III. Bereitstellen einer Zusammensetzung (C), enthaltend
h) mindestens eine Peroxidverbindung, die bevorzugt Wasserstoffperoxid ist, wobei die Zusammensetzung (C) bevorzugt einen pH-Wert im Bereich von 2,5 bis 6,5, bevorzugt 3,0 - 5,5, besonders bevorzugt 3,5 bis 5,0, aufweist, jeweils gemessen bei 20°C,
IV. Vermischen der Zusammensetzungen (A), (B) und (C) miteinander, direkt anschließend
V. Auftragen der Mischung aus (A), (B) und (C) auf die keratinischen Fasern, insbesondere auf das Humanhaar, und
VI. Ausspülen nach einer Einwirkzeit von 0,1 bis 60 Minuten, bevorzugt 1 bis 45 Minuten, besonders bevorzugt 10 bis 30 Minuten,
VII. ggf, weitere Haarbehandlungen, wie Verformen, Konditionieren und/oder Trocknen.

12. Zusammensetzung (A), enthaltend
- mindestens eine gesättigte Dicarbonsäure mit 2 bis 10 Kohlenstoffatomen in einer Gesamtmenge von 2 bis 20 Gew.-%, bevorzugt 5 bis 15 Gew.-%, besonders bevorzugt 8 bis 12 Gew.-%, jeweils umgerechnet auf die undissoziierte Dicarbonsäure und bezogen auf das Gewicht der Zusammensetzung (A), wobei die Dicarbonsäure bevorzugt ausgewählt ist aus Bernsteinsäure, Oxalsäure, Malonsäure, Adipinsäure, Pimelinsäure, Korksäure, Azelainsäure, Sebacinsäure, sowie Mischungen dieser Säuren, wobei Bernsteinsäure außerordentlich bevorzugt ist, weiterhin
- mindestens eine Aminosäure der Formel (VI) und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A), wobei bevorzugt mindestens eine der Aminosäuren Arginin, Histidin oder Lysin und/oder ein Salz hiervon in einer Gesamtmenge von 0,4 bis 7,0 Gew.-%, bevorzugt 0,8 bis 5,0 Gew.-%, besonders bevorzugt 1,5 bis 4,0 Gew.-%, jeweils umgerechnet auf die undissoziierte Aminosäure und bezogen auf das Gewicht der Zusammensetzung (A) enthalten ist, und
- Wasser, bevorzugt in einer Menge von 50 - 92 Gew.-%, besonders bevorzugt 60 - 87 Gew.-% und außerordentlich bevorzugt von 65 bis 80 Gew.-%, jeweils bezogen auf das Gewicht der Zusammensetzung (A),
- optional weiterhin mindestens ein Polymer A, das mindestens zehn konstitutive Einheiten der Formel (I) aufweist, 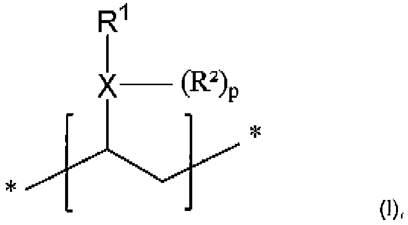 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht,
wobei das Polymer A keine permanent-ionischen konstitutiven Einheiten enthält,
wobei bevorzugt das mindestens eine Polymer A in einer Gesamtmenge von 0,5 bis 14 Gew.-%, bevorzugt 1,0 bis 11 Gew.-%, besonders bevorzugt 2,0 bis 10 Gew.-%, jeweils bezogen auf das Gewicht der Zusammensetzung (A), enthalten ist,
wobei die Zusammensetzung (A) bevorzugt einen pH-Wert im Bereich von 3,5 bis 7,1, bevorzugt 4,5 - 6,5, besonders bevorzugt 5,0 bis 6,0, aufweist, jeweils gemessen bei 20°C.

13. Verfahren zur oxidativen Färbung und/oder Aufhellung keratinischer Fasern, insbesondere von Humanhaar, das folgende Verfahrensschritte umfasst:
I. Bereitstellen einer Zusammensetzung (AB), enthaltend
a) mindestens eine gesättigte Dicarbonsäure mit 2 bis 10 Kohlenstoffatomen und/oder mindestens ein Salz dieser Säure(n),
b) mindestens eine Aminosäure der Formel (VI), 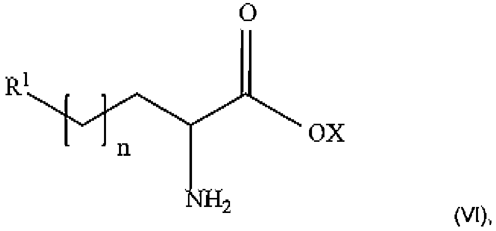 worin X für ein Wasserstoffatom oder ein ein- oder zweiwertiges Kation steht;
n für null, 1, 2 oder 3 steht;
R¹ für einen Rest steht, der ausgewählt ist aus einer Aminogruppe, einer Guanidin-Gruppe, einer (1*H*-Imidazol-4-yl)-Gruppe, einer Carbonsäureamid-Gruppe -CONH₂, einer 1*H*-Indol-3-yl-Gruppe, einer Thiol-Gruppe -SH und einer Methylsulfanyl-Gruppe -SCH₃, oder mindestens ein Salz dieser Aminosäure,
wobei die Aminosäure der Formel (VI) bevorzugt ausgewählt ist aus Arginin, Lysin, Histidin sowie Mischungen hiervon, besonders bevorzugt Mischungen aus Arginin und Lysin, oder mindestens einem Salz dieser Aminosäuren,
c) optional weiterhin mindestens eine Substanz, die ausgewählt ist aus
- Verbindungen der allgemeinen Formel (III), 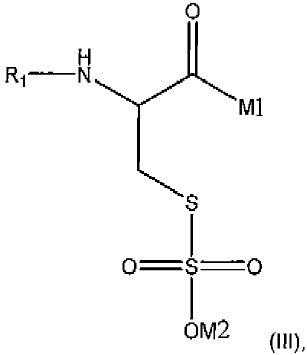 wobei
R1 für ein Wasserstoffatom oder für ein Strukturelement der Formel (IV) steht 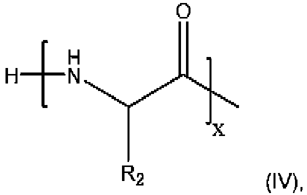 wobei
x für eine ganze Zahl von 1 bis 100 steht,
der Rest R2 in jedem der Strukturelemente der Formel (IV) jeweils unabhängig vom vorangegangenen Strukturelement der Formel (IV) gewählt werden kann,
R2 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-Imidazol-4-ylmethyl-Gruppe, eine 1H-Indol-3-ylmethyl-Gruppe oder eine (Sulfosulfanyl)methyl-Gruppe,
M1 steht für die Gruppierung -OM2 oder für ein Strukturelement der Formel (V) 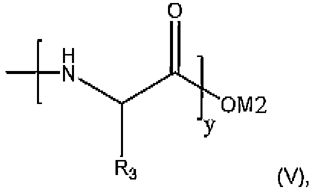 wobei y für eine ganze Zahl von 1 bis 100 steht,
der Rest R3 in jedem der Strukturelemente der Formel (V) jeweils unabhängig vom vorangegangenen Strukturelement der Formel (V) gewählt werden kann,
R3 steht für ein Wasserstoffatom, eine Methylgruppe, eine Isopropylgruppe, eine 2-Methylpropylgruppe, eine 1-Methyl-propylgruppe, eine Benzylgruppe, eine 4-Hydroxybenzylgruppe, eine Hydroxymethylgruppe, eine 1-Hydroxyethylgruppe, eine 4-Aminobutylgruppe, eine 3-Carbamimidamidopropyl-Gruppe, eine 2-Carboxyethyl-Gruppe, eine Carboxymethyl-Gruppe, eine 2-Carbamoylethyl-Gruppe, eine Carbamoylmethyl-Gruppe, eine Sulfanylmethyl-Gruppe, eine 2-(Methylsulfanyl)ethyl-Gruppe, eine 1H-[midazol-4-ylmethyl-Gruppe, eine 1H-Indol-3-ylmethyl-Gruppe oder eine (Sulfosulfanyl)methyl-Gruppe,
M2 steht für ein Wasserstoffatom, ein Äquivalent eines ein oder mehrwertigen Kations oder ein Ammoniumion (NH₄)⁺, und
- Polymeren A, die mindestens zehn konstitutive Einheiten der Formel (I) aufweisen, 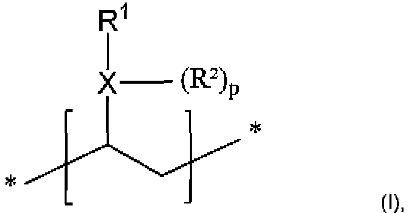 worin
- X für Stickstoff oder Sauerstoff steht und
- R¹ und R² jeweils unabhängig voneinander für Wasserstoff oder eine C2-C10-Acylgruppe stehen oder R¹ und R² zusammen mit X einen fünf- oder sechsgliedrigen, gesättigten oder ungesättigten Ring bilden, der gegebenenfalls weitere Heteroatome enthält, die bevorzugt ausgewählt sind aus N und O, und/oder gegebenenfalls mit mindestens einer C1 - C6-Alkylgruppe und/oder mit mindestens einer funktionellen Gruppe substituiert ist, und
- p = 0 ist, wenn X für Sauerstoff steht und p = 1 ist, wenn X für Stickstoff steht, wobei das Polymer A keine permanent-ionischen konstitutiven Einheiten enthält,
d) mindestens ein Alkalisierungsmittel, ausgewählt aus Ammoniumhydroxid, Monoethanolamin und Natriumsilikaten sowie Mischungen hiervon,
e) Wasser und
f) ggf. mindestens ein Oxidationsfarbstoffvorprodukt und/oder mindestens einen direktziehenden Farbstoff,
II. Bereitstellen einer Zusammensetzung (C), enthaltend
g) mindestens eine Peroxidverbindung, die bevorzugt Wasserstoffperoxid ist,
III. Vermischen der Zusammensetzungen (AB) und (C) miteinander, direkt anschließend
IV. Auftragen der Mischung aus (AB) und (C) auf die keratinischen Fasern, insbesondere auf das Humanhaar, und
V. Ausspülen nach einer Einwirkzeit von 0,1 bis 60 Minuten, bevorzugt 1 bis 45 Minuten, besonders bevorzugt 10 bis 30 Minuten,
VI. ggf. weitere Haarbehandlungen, wie Verformen, Konditionieren und/oder Trocknen.

14. Verfahren gemäß Anspruch 11 oder 13, **dadurch gekennzeichnet, dass** die mindestens eine gesättigte Dicarbonsäure mit 2 bis 10 Kohlenstoffatomen ausgewählt ist aus Bernsteinsäure.

15. Färbe- oder Blondiermittel gemäß einem der Ansprüche 8 - 9 oder Verfahren gemäß einem der Ansprüche 11, 13 oder 14 oder Zusammensetzung (A) gemäß Anspruch 12, **dadurch gekennzeichnet, dass** das mindestens eine Polymer (A), das mindestens zehn konstitutive Einheiten der Formel (I) aufweist und keine permanent-ionischen konstitutiven Einheiten enthält, ausgewählt ist aus Polyvinylpyrrolidon, Polyvinylalkohol sowie aus Mischungen hiervon, wobei Polyvinylpyrrolidon besonders bevorzugt ist.

## Claims

1. An oxidative coloring or bleaching agent for keratin fibers, in particular for human hair, containing
a) at least one saturated dicarboxylic acid having 2 to 10 carbon atoms and/or at least one salt of said acid(s),
b) at least one amino acid of formula (VI) 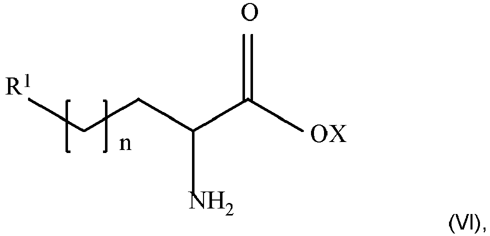 in which
X represents a hydrogen atom or a monovalent or divalent cation;
n represents zero, 1, 2 or 3;
R¹ represents a radical selected from an amino group, a guanidine group, a (1H-imidazol-4-yl) group, a carboxamide group -CONH₂, a 1H-indol-3-yl group, a thiol group -SH and a methylsulfanyl group -SCH₃, or at least one salt of said amino acid,
c) also at least one alkalizing agent, selected from ammonium hydroxide, monoethanolamine and sodium silicates, and mixtures thereof,
d) optionally at least one oxidation dye precursor and/or at least one substantive dye,
e) water, and
f) at least one peroxide compound.

2. The coloring or bleaching agent according to claim 1, **characterized in that** the at least one saturated dicarboxylic acid having 2 to 10 carbon atoms is selected from succinic acid, oxalic acid, malonic acid, adipic acid, pimelic acid, suberic acid, azelaic acid, sebacic acid, and mixtures of said acids, preferably selected from succinic acid.

3. The coloring or bleaching agent according to claim 1 or 2, **characterized in that** the at least one saturated dicarboxylic acid having 2 to 10 carbon atoms is contained in a total amount of 0.2 to 4% by weight, preferably 0.33 to 3% by weight, particularly preferably 0.5 to 2% by weight, in each case converted to the undissociated acid and based on the weight of the coloring or bleaching agent.

4. The coloring or bleaching agent according to any of claims 1 to 3, **characterized in that** the at least one amino acid of formula (VI) is selected from arginine, lysine, histidine, asparagine, glutamine, cysteine, methionine, tryptophan and mixtures thereof, particularly preferably mixtures of arginine and lysine.

5. The coloring or bleaching agent according to any of claims 1 to 4, **characterized in that** the at least one amino acid of formula (VI) is contained in a total amount of 0.05 to 3% by weight, preferably 0.1 to 2% by weight, particularly preferably 0.2 to 1.2% by weight, in each case converted to the undissociated acid and based on the weight of the coloring or bleaching agent.

6. The coloring or bleaching agent according to any of claims 1 to 5, **characterized in that** it contains at least one compound of general formula (III) 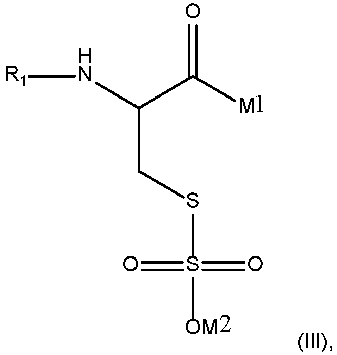 wherein
R1 represents a hydrogen atom or a structural element of formula (IV) 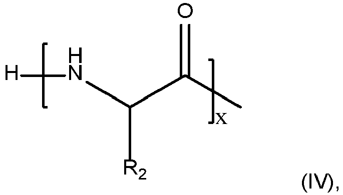 wherein
x represents an integer from 1 to 100,
the radical R2 in each of the structural elements of formula (IV) can in each case be selected independently of the preceding structural element of formula (IV),
R2 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1H-indol-3-ylmethyl group or a (sulfosulfanyl)methyl group, M1 represents the group -OM2 or a structural element of formula (V) 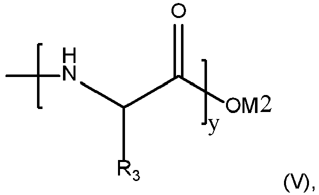 wherein
y represents an integer from 1 to 100,
the radical R3 in each of the structural elements of formula (V) can in each case be selected independently of the preceding structural element of formula (V),
R3 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1H-imidazol-4-ylmethyl group, a 1H-indol-3-ylmethyl group or a (sulfosulfanyl)methyl group, M2 represents a hydrogen atom, an equivalent of a monovalent or polyvalent cation or an ammonium ion (NH4)⁺,
wherein preferably one or more compounds of the above formula (III) are contained in a total amount of 0.001 to 2.5% by weight, more preferably 0.01 to 1.0% by weight and particularly preferably 0.02 to 0.1% by weight, in each case based on the weight of the coloring or bleaching agent according to the invention.

7. The coloring or bleaching agent according to any of claims 1 to 6, **characterized in that** it contains hydrogen peroxide as the peroxide compound.

8. The coloring or bleaching agent according to any of claims 1 to 7, **characterized in that** it contains at least one polymer A which has at least ten constituent units of formula (I) 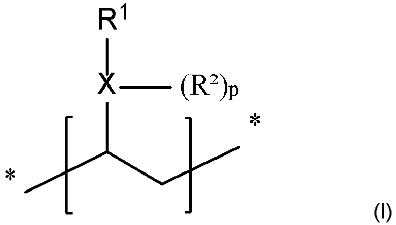 in which
- X represents nitrogen or oxygen and
- R¹ and R² in each case independently of one another represent hydrogen or a C2-C10 acyl group or R¹ and R² together with X form a five-membered or six-membered, saturated or unsaturated ring which optionally contains further heteroatoms preferably selected from N and O, and/or is optionally substituted with at least one C1-C6 alkyl group and/or with at least one functional group, and
- p = 0 when X represents oxygen and p = 1 when X represents nitrogen,
the polymer A containing no permanently ionic constituent units,
wherein the at least one polymer A having at least ten constituent units of formula (I) is preferably contained in a total amount of 0.2 to 5% by weight, particularly preferably 0.5 to 3% by weight, extremely preferably 1.0 to 2.3% by weight, in each case based on the weight of the coloring or bleaching agent.

9. The coloring or bleaching agent according to claim 8, **characterized in that** the at least one polymer A having at least ten constituent units of formula (I) is selected from polymers which comprise 98-100 mol% constituent units of formula (la) 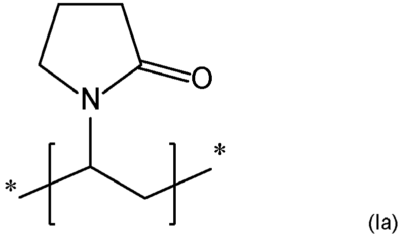 and have a degree of polymerization in the range from 40 to 1000, preferably 100 to 800, particularly preferably 350 to 650, the polymer A containing no permanently ionic constituent units.

10. A method for the oxidative coloring and/or lightening of keratin fibers, in particular of human hair, in which a coloring or bleaching agent according to one of claims 1 to 9 is applied to the keratin fibers, in particular to the human hair, and is rinsed out again after a leave-in time of 0.1 to 60 minutes, preferably 1 to 45 minutes, particularly preferably 10 to 30 minutes.

11. A method for the oxidative coloring and/or lightening of keratin fibers, in particular of human hair, which comprises the following method steps:
I. providing a composition (A), containing
a) at least one saturated dicarboxylic acid having 2 to 10 carbon atoms and/or at least one salt of said acid(s),
b) at least one amino acid of formula (VI) 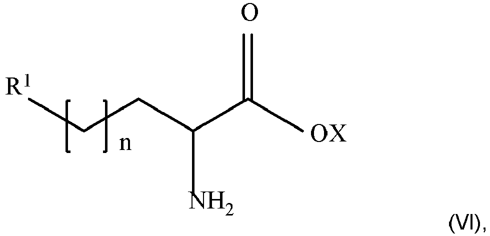 in which
X represents a hydrogen atom or a monovalent or divalent cation;
n represents zero, 1, 2 or 3;
R¹ represents a radical selected from an amino group, a guanidine group, a (1H-imidazol-4-yl) group, a carboxamide group -CONH₂, a 1H-indol-3-yl group, a thiol group -SH and a methylsulfanyl group -SCH₃, or at least one salt of said amino acid,
c) water, and
d) optionally also at least one substance selected from
- compounds of general formula (III) 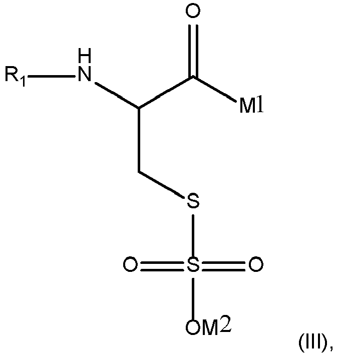 wherein
R1 represents a hydrogen atom or a structural element of formula (IV) 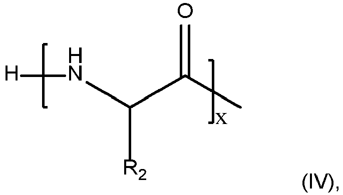 wherein
x represents an integer from 1 to 100,
the radical R2 in each of the structural elements of formula (IV) can in each case be selected independently of the preceding structural element of formula (IV),
R2 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1 H-imidazol-4-ylmethyl group, a 1 H-indol-3-ylmethyl group or a (sulfosulfanyl)methyl group,
M1 represents the group -OM2 or a structural element of formula (V) 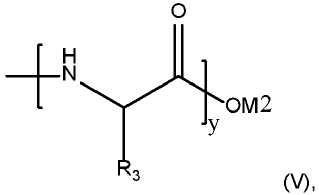 wherein
y represents an integer from 1 to 100,
the radical R3 in each of the structural elements of formula (V) can in each case be selected independently of the preceding structural element of formula (V),
R3 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1 H-imidazol-4-ylmethyl group, a 1 H-indol-3-ylmethyl group or a (sulfosulfanyl)methyl group,
M2 represents a hydrogen atom, an equivalent of a monovalent or polyvalent cation or an ammonium ion (NH₄)⁺, and
- polymers A which have at least ten constituent units of formula (I) 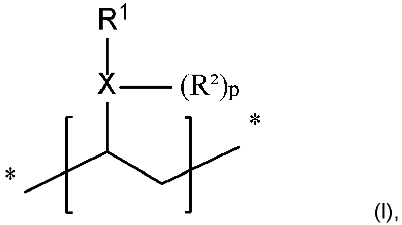 in which
- X represents nitrogen or oxygen and
- R¹ and R² in each case independently of one another represent hydrogen or a C2-C10 acyl group or R¹ and R² together with X form a five-membered or six-membered, saturated or unsaturated ring which optionally contains further heteroatoms preferably selected from N and O, and/or is optionally substituted with at least one C1-C6 alkyl group and/or with at least one functional group, and
- p = 0 when X represents oxygen and p = 1 when X represents nitrogen,
the polymer A containing no permanently ionic constituent units,
wherein the composition (A) preferably has a pH in the range from 3.5 to 7.1, preferably 4.5 to 6.5, particularly preferably 5.0 to 6.0, in each case measured at 20°C,
II. providing a composition (B), containing
e) at least one alkalizing agent, selected from ammonium hydroxide, monoethanolamine and sodium silicates, and mixtures thereof,
f) optionally water, and
g) optionally at least one oxidation dye precursor and/or at least one substantive dye,
wherein the composition (B) preferably has a pH in the range from 6.5 to 11.0, preferably 8 to 10.5, particularly preferably 8.5 to 10.0, in each case measured at 20°C,
III. providing a composition (C), containing
h) at least one peroxide compound, which is preferably hydrogen peroxide,
wherein the composition (C) preferably has a pH in the range from 2.5 to 6.5, preferably 3.0 to 5.5, particularly preferably 3.5 to 5.0, in each case measured at 20°C,
IV. mixing compositions (A), (B) and (C) with one another, then immediately
V. applying the mixture of (A), (B) and (C) to the keratin fibers, in particular to the human hair, and
VI. rinsing out after a leave-in time of 0.1 to 60 minutes, preferably 1 to 45 minutes, particularly preferably 10 to 30 minutes,
VII. optionally further hair treatments, such as styling, conditioning and/or drying.

12. A composition (A), containing
- at least one saturated dicarboxylic acid having 2 to 10 carbon atoms in a total amount of 2 to 20% by weight, preferably 5 to 15% by weight, particularly preferably 8 to 12% by weight, in each case converted to the undissociated acid and based on the weight of the composition (A), the dicarboxylic acid preferably being selected from succinic acid, oxalic acid, malonic acid, adipic acid, pimelic acid, suberic acid, azelaic acid, sebacic acid, and mixtures of said acids, extreme preference being given to succinic acid, and
- at least one amino acid of formula (VI) and/or a salt thereof in a total amount of 0.4 to 7.0% by weight, preferably 0.8 to 5.0% by weight, particularly preferably 1.5 to 4.0% by weight, in each case converted to the undissociated acid and based on the weight of the composition (A), wherein preferably at least one of the amino acids arginine, histidine or lysine and/or a salt thereof is contained in a total amount of 0.4 to 7.0% by weight, preferably 0.8 to 5.0% by weight, particularly preferably 1.5 to 4.0% by weight, in each case converted to the undissociated acid and based on the weight of the composition (A), and
- water, preferably in an amount of 50 to 92% by weight, particularly preferably 60 to 87% by weight and extremely preferably 65 to 80% by weight, in each case based on the weight of the composition (A),
- optionally also at least one polymer A which has at least ten constituent units of formula (I) 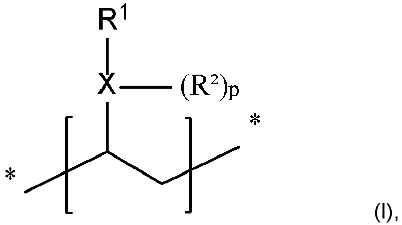 in which
- X represents nitrogen or oxygen and
- R¹ and R² in each case independently of one another represent hydrogen or a C2-C10 acyl group or R¹ and R² together with X form a five-membered or six-membered, saturated or unsaturated ring which optionally contains further heteroatoms preferably selected from N and O, and/or is optionally substituted with at least one C1-C6 alkyl group and/or with at least one functional group, and
- p = 0 when X represents oxygen and p = 1 when X represents nitrogen,
the polymer A containing no permanently ionic constituent units,
wherein preferably the at least one polymer A is contained in a total amount of 0.5 to 14% by weight, preferably 1.0 to 11% by weight, particularly preferably 2.0 to 10% by weight, in each case based on the weight of the composition (A),
wherein the composition (A) preferably has a pH in the range from 3.5 to 7.1, preferably 4.5 to 6.5, particularly preferably 5.0 to 6.0, in each case measured at 20°C.

13. A method for the oxidative coloring and/or lightening of keratin fibers, in particular of human hair, which comprises the following method steps:
I. providing a composition (AB), containing
a) at least one saturated dicarboxylic acid having 2 to 10 carbon atoms and/or at least one salt of said acid(s),
b) at least one amino acid of formula (VI) 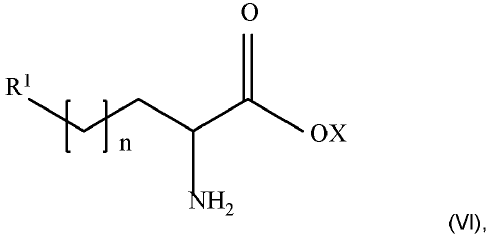 in which
X represents a hydrogen atom or a monovalent or divalent cation;
n represents zero, 1, 2 or 3;
R¹ represents a radical selected from an amino group, a guanidine group, a (1H-imidazol-4-yl) group, a carboxamide group -CONH₂, a 1H-indol-3-yl group, a thiol group -SH and a methylsulfanyl group -SCH₃, or at least one salt of said amino acid, the amino acid of formula (VI) preferably being selected from arginine, lysine, histidine and mixtures thereof, particularly preferably mixtures of arginine and lysine, or at least one salt of said amino acids,
c) optionally also at least one substance selected from
- compounds of general formula (III) 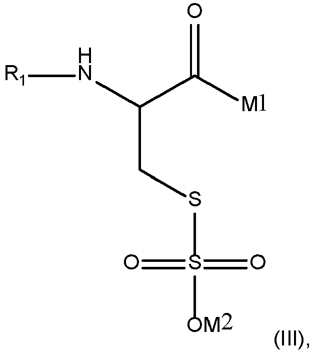 wherein
R1 represents a hydrogen atom or a structural element of formula (IV) 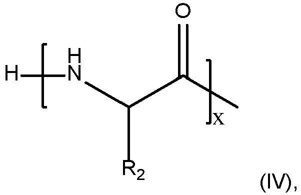 wherein
x represents an integer from 1 to 100,
the radical R2 in each of the structural elements of formula (IV) can in each case be selected independently of the preceding structural element of formula (IV),
R2 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1 H-imidazol-4-ylmethyl group, a 1 H-indol-3-ylmethyl group or a (sulfosulfanyl)methyl group,
M1 represents the group -OM2 or a structural element of formula (V) 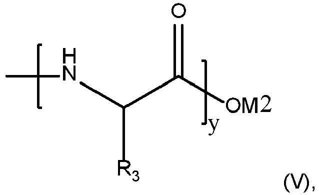 wherein
y represents an integer from 1 to 100,
the radical R3 in each of the structural elements of formula (V) can in each case be selected independently of the preceding structural element of formula (V),
R3 represents a hydrogen atom, a methyl group, an isopropyl group, a 2-methylpropyl group, a 1-methylpropyl group, a benzyl group, a 4-hydroxybenzyl group, a hydroxymethyl group, a 1-hydroxyethyl group, a 4-aminobutyl group, a 3-carbamimidamidopropyl group, a 2-carboxyethyl group, a carboxymethyl group, a 2-carbamoylethyl group, a carbamoylmethyl group, a sulfanylmethyl group, a 2-(methylsulfanyl)ethyl group, a 1 H-imidazol-4-ylmethyl group, a 1 H-indol-3-ylmethyl group or a (sulfosulfanyl)methyl group,
M2 represents a hydrogen atom, an equivalent of a monovalent or polyvalent cation or an ammonium ion (NH₄)⁺, and
- polymers A which have at least ten constituent units of formula (I) 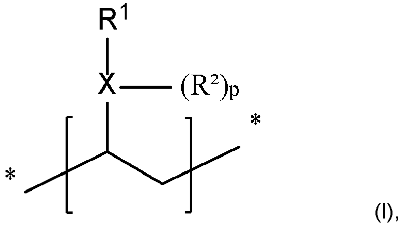 in which
- X represents nitrogen or oxygen and
- R¹ and R² in each case independently of one another represent hydrogen or a C2-C10 acyl group or R¹ and R² together with X form a five-membered or six-membered, saturated or unsaturated ring which optionally contains further heteroatoms preferably selected from N and O, and/or is optionally substituted with at least one C1-C6 alkyl group and/or with at least one functional group, and
- p = 0 when X represents oxygen and p = 1 when X represents nitrogen,
the polymer A containing no permanently ionic constituent units,
d) at least one alkalizing agent, selected from ammonium hydroxide, monoethanolamine and sodium silicates, and mixtures thereof,
e) water, and
f) optionally at least one oxidation dye precursor and/or at least one substantive dye,
II. providing a composition (C), containing
g) at least one peroxide compound, which is preferably hydrogen peroxide,
III. mixing compositions (AB) and (C) with one another, then immediately
IV. applying the mixture of (AB) and (C) to the keratin fibers, in particular to the human hair, and
V. rinsing out after a leave-in time of 0.1 to 60 minutes, preferably 1 to 45 minutes, particularly preferably 10 to 30 minutes,
VI. optionally further hair treatments, such as styling, conditioning and/or drying.

14. The method according to claim 11 or 13, **characterized in that** the at least one saturated dicarboxylic acid having 2 to 10 carbon atoms is selected from succinic acid.

15. The coloring or bleaching agent according to any of claims 8 to 9 or the method according to any of claims 11, 13 or 14 or the composition (A) according to claim 12, **characterized in that** the at least one polymer (A) which has at least ten constituent units of formula (I) and which contains no permanently ionic constituent units is selected from polyvinylpyrrolidone, polyvinyl alcohol and mixtures thereof, particular preference being given to polyvinylpyrrolidone.

## Revendications

1. Produit de coloration et de décoloration par oxydation pour fibres kératiniques, en particulier pour cheveux humains, contenant
a) au moins un acide dicarboxylique saturé ayant 2 à 10 atomes de carbone et/ou au moins un sel de cet/ces acide(s),
b) au moins un acide aminé de formule (VI) 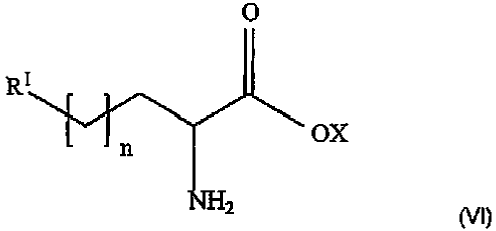 dans laquelle
X représente un atome d'hydrogène ou un cation monovalent ou divalent ;
n est égal à zéro, 1, 2 ou 3 ;
R¹ représente un radical choisi parmi un groupe amino, un groupe guanidine, un groupe (1H-imidazol-4-yl), un groupe amide d'acide carboxylique -CONH₂, un groupe 1H-indol-3-yl, un groupe thiol -SH et un groupe méthylsulfanyle -SCH₃, ou au moins un sel de cet acide aminé,
c) en outre au moins un produit d'alcalinisation choisi parmi l'hydroxyde d'ammonium, la monoéthanolamine et les silicates de sodium ainsi que des mélanges de ceux-ci,
d) éventuellement au moins un précurseur de colorant d'oxydation et/ou au moins un colorant direct,
e) de l'eau, et
f) au moins un composé peroxyde.

2. Produit de coloration ou de décoloration selon la revendication 1, **caractérisé en ce que** l'au moins un acide dicarboxylique saturé ayant 2 à 10 atomes de carbone est choisi parmi l'acide succinique, l'acide oxalique, l'acide malonique, l'acide adipique, l'acide pimélique, l'acide subérique, l'acide azélaïque, l'acide sébacique, ainsi que des mélanges de ces acides, choisi de préférence parmi l'acide succinique.

3. Produit de coloration et de décoloration selon la revendication 1 ou 2, **caractérisé en ce que** l'au moins un acide dicarboxylique saturé ayant 2 à 10 atomes de carbone est présent en une quantité totale de 0,2 à 4 % en poids, de préférence de 0,33 à 3 % en poids, de manière particulièrement préférée de 0,5 à 2 % en poids, converti dans chaque cas en acide dicarboxylique non dissocié et par rapport au poids du produit de coloration ou de décoloration.

4. Produit de coloration ou de décoloration selon l'une des revendications 1 à 3, **caractérisé en ce que** l'au moins un acide aminé de formule (VI) est choisi parmi l'arginine, la lysine, l'histidine, l'asparagine, la glutamine, la cystéine, la méthionine, le tryptophane ainsi que des mélanges de ceux-ci, de manière particulièrement préférée des mélanges d'arginine et de lysine.

5. Produit de coloration ou de décoloration selon l'une des revendications 1 à 4, **caractérisé en ce que** l'au moins un acide aminé de formule (VI) est présent en une quantité totale de 0,05 à 3 % en poids, de préférence de 0,1 à 2 % en poids, de manière particulièrement préférée de 0,2 à 1,2 % en poids, converti dans chaque cas en acide aminé non dissocié et par rapport au poids du produit de coloration ou de décoloration.

6. Produit de coloration ou de décoloration selon l'une des revendications 1 à 5, **caractérisé en ce qu'**au moins un composé de formule générale (III) est présent, 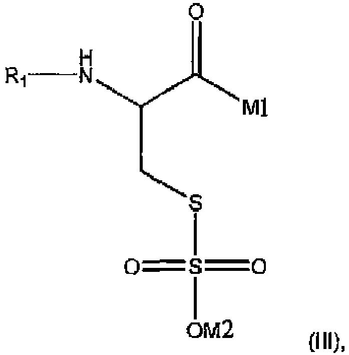 dans laquelle
R¹ représente un atome d'hydrogène ou un élément de structure de formule (IV) 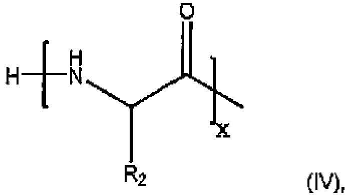 dans laquelle
x représente un nombre entier de 1 à 100,
le radical R₂ dans chacun des éléments de structure de formule (IV) peut être choisi dans chaque cas indépendamment de l'élément de structure précédent de formule (IV),
R₂ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M1 représente le groupement -OM2 ou un élément de structure de formule (V) 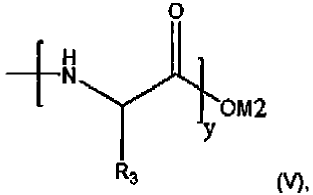 dans laquelle
y représente un nombre entier de 1 à 100,
le radical R₃ dans chacun des éléments de structure de formule (V) peut être choisi dans chaque cas indépendamment de l'élément de structure précédent de formule (V),
R₃ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation monovalent ou polyvalent ou un ion ammonium (NH₄)⁺,
un ou plusieurs composés de la formule (III) susmentionnée étant de préférence présents en une quantité totale de 0,001 à 2,5 % en poids, de manière davantage préférée de 0,01 à 1,0 % en poids et de manière particulièrement préférée de 0,02 à 0,1 % en poids, dans chaque cas par rapport au poids du produit de coloration ou de décoloration selon l'invention.

7. Produit de coloration ou de décoloration selon l'une des revendications 1 à 6, **caractérisé en ce qu'**un peroxyde d'hydrogène est présent comme composé peroxyde.

8. Produit de coloration ou de décoloration selon l'une des revendications 1 à 7, **caractérisé en ce qu'**au moins un polymère A est présent et présente au moins dix unités constitutives de formule (I), 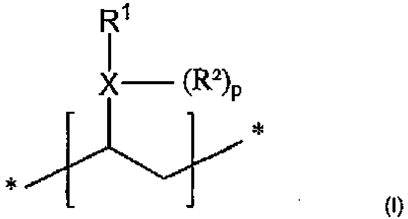 dans laquelle
- X représente l'azote ou l'oxygène, et
- R¹ et R² représentent chacun indépendamment l'hydrogène ou un groupe acyle en C2-C10 ou R¹ et R² forment, avec X, un cycle à cinq ou six chaînons, saturé ou insaturé, qui contient éventuellement d'autres hétéroatomes qui sont de préférence choisis parmi N et O, et/ou éventuellement substitués par au moins un groupe alkyle en C1-C6 et/ou par au moins un groupe fonctionnel, et
- p = 0 si X représente l'oxygène et p = 1 si X représente l'azote, le polymère A
ne contenant pas d'unités constitutives ioniques permanentes,
l'au moins un polymère A présentant au moins dix unités constitutives de formule (I) étant présent de préférence en une quantité totale de 0,2 à 5 % en poids, de manière particulièrement préférée de 0,5 à 3 % en poids, de manière préférée entre toutes de 1,0 à 2,3 % en poids, dans chaque cas par rapport au poids du produit de coloration ou de décoloration.

9. Produit de coloration ou de décoloration selon la revendication 8, **caractérisé en ce que** l'au moins un polymère A présentant au moins dix unités constitutives de formule (I) est choisi parmi les polymères qui présentent des unités constitutives de formule (la) à raison de 98 à 100 % en moles 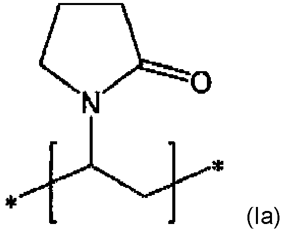 et ont un degré de polymérisation situé dans la plage de 40 à 1000, de préférence de 100 à 800, de manière particulièrement préférée de 350 à 650, le polymère A ne contenant pas d'unités constitutives ioniques permanentes.

10. Procédé de coloration et/ou d'éclaircissement par oxydation de fibres kératiniques, en particulier de cheveux humains, dans lequel un produit de coloration ou de décoloration selon l'une des revendications 1 à 9 est appliqué sur les fibres keratiniques, en particulier sur les cheveux humains, et est ensuite rincé après une durée d'action de 0,1 à 60 minutes, de préférence de 1 à 45 minutes, de manière particulièrement préférée de 10 à 30 minutes.

11. Procédé de coloration et/ou d'éclaircissement par oxydation de fibres kératiniques, en particulier de cheveux humains, comprenant les étapes de procédé suivantes :
I. préparation d'une composition (A) contenant
a) au moins un acide dicarboxylique saturé ayant 2 à 10 atomes de carbone et/ou au moins un sel de cet/ces acide(s),
b) au moins un acide aminé de formule (VI) 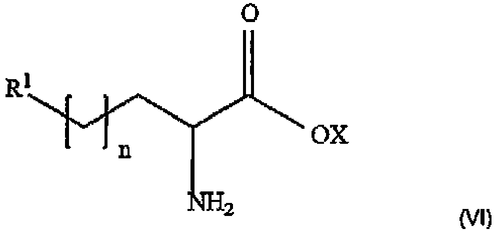 dans laquelle
X représente un atome d'hydrogène ou un cation monovalent ou divalent ;
n est égal à zéro, 1, 2 ou 3 ;
R¹ représente un radical choisi parmi un groupe amino, un groupe guanidine, un groupe (1H-imidazol-4-yl), un groupe amide d'acide carboxylique -CONH₂, un groupe 1H-indol-3-yl, un groupe thiol -SH et un groupe méthylsulfanyle -SCH₃, ou au moins un sel de cet acide aminé,
c) de l'eau, et
d) éventuellement en outre au moins une substance choisie parmi
- des composés de formule générale (III), 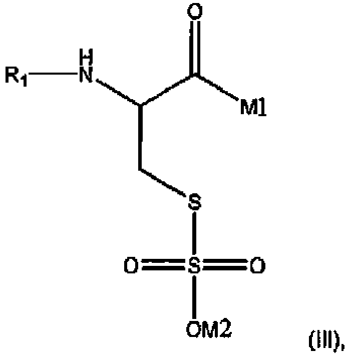 dans laquelle
R¹ représente un atome d'hydrogène ou un élément de structure de formule (IV) 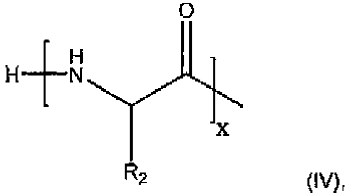 dans laquelle
x représente un nombre entier de 1 à 100,
le radical R₂ dans chacun des éléments de structure de formule (IV) peut être choisi dans chaque cas indépendamment de l'élément de structure précédent de formule (IV),
R₂ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M1 représente le groupement -OM2 ou un élément de structure de formule (V) 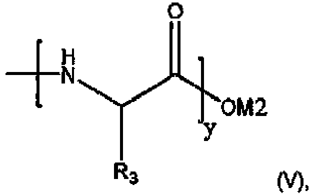 dans laquelle
y représente un nombre entier de 1 à 100,
le radical R₃ dans chacun des éléments de structure de formule (V) peut être choisi dans chaque cas indépendamment de l'élément de structure précédent de formule (V),
R₃ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation monovalent ou polyvalent ou un ion ammonium (NH₄)⁺, et
- les polymères A qui présentent au moins dix unités constitutives de formule (I), 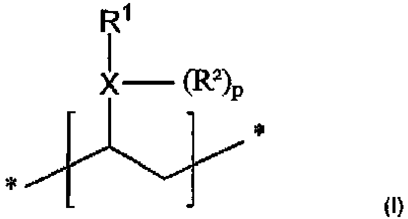 dans laquelle
- X représente l'azote ou l'oxygène, et
- R¹ et R² représentent chacun indépendamment l'hydrogène ou un groupe acyle en C2-C10 ou R¹ et R² forment, avec X, un cycle à cinq ou six chaînons, saturé ou insaturé, qui contient éventuellement d'autres hétéroatomes qui sont de préférence choisis parmi N et O, et/ou éventuellement substitués par au moins un groupe alkyle en C1-C6 et/ou par au moins un groupe fonctionnel, et
- p = 0 si X représente l'oxygène et p = 1 si X représente l'azote,
le polymère A ne contenant pas d'unités constitutives ioniques permanentes,
la composition (A) présentant de préférence un pH dans une plage de 3,5 à 7,1, de préférence de 4,5 à 6,5, de manière particulièrement préférée de 5,0 à 6,0, mesuré dans chaque cas à 20 °C,
II. préparation d'une composition (B) contenant
e) au moins un produit d'alcalinisation choisi parmi l'hydroxyde d'ammonium, la monoéthanolamine et les silicates de sodium ainsi que des mélanges de ceux-ci,
f) éventuellement de l'eau, et
g) éventuellement au moins un précurseur de colorant d'oxydation et/ou au moins un colorant direct,
la composition (B) ayant de préférence un pH dans une plage de 6,5 à 11,0, de préférence de 8 à 10,5, de manière particulièrement préférée de 8,5 à 10,0, mesuré dans chaque cas à 20 °C,
III. préparation d'une composition (C) contenant
h) au moins un composé peroxyde, qui est de préférence un peroxyde d'hydrogène, la composition (C) présentant de préférence un pH dans une plage de 2,5 à 6,5, de préférence de 3,0 à 5,5, de manière particulièrement préférée de 3,5 à 5,0, mesuré dans chaque cas à 20 °C,
IV. mélange des compositions (A), (B) et (C) les unes avec les autres, immédiatement après
V. application du mélange de (A), (B) et (C) sur les fibres kératiniques, en particulier sur les cheveux humains, et
VI. rinçage après une durée d'action de 0,1 à 60 minutes, de préférence de 1 à 45 minutes, de manière particulièrement préférée de 10 à 30 minutes,
VII. éventuellement d'autres traitements capillaires tels que le brushing, le conditionnement et/ou le séchage.

12. Composition (A), contenant
- au moins un acide dicarboxylique saturé ayant 2 à 10 atomes de carbone en une quantité totale de 2 à 20 % en poids, de préférence de 5 à 15 % en poids, de manière particulièrement préférée de 8 à 12 % en poids, converti dans chaque cas en acide dicarboxylique non dissocié et par rapport au poids de la composition (A), l'acide dicarboxylique étant de préférence choisi parmi l'acide succinique, l'acide oxalique, l'acide malonique, l'acide adipique, l'acide pimélique, l'acide subérique, l'acide azélaïque, l'acide sébacique ainsi que des mélanges de ces acides, l'acide succinique étant préféré entre tous
- au moins un acide aminé de formule (VI) et/ou un sel de celui-ci en une quantité totale de 0,4 à 7,0 % en poids, de préférence de 0,8 à 5,0 % en poids, de manière particulièrement préférée 1,5 à 4,0 % en poids, converti dans chaque cas en acide aminé non dissocié et par rapport au poids de la composition (A), de préférence au moins l'un des éléments que sont les acides aminés l'arginine, l'histidine ou la lysine et/ou un sel de ceux-ci en une quantité totale de 0,4 à 7,0 % en poids, de préférence de 0,8 à 5,0 % en poids, de manière particulièrement préférée de 1,5 à 4,0 % en poids, convertis dans chaque cas en acide aminé non dissocié et par rapport du poids de la composition (A), et
- de l'eau, de préférence en une quantité de 50 à 92 % en poids, de préférence de 60 à 87 % en poids et de manière préférée entre toutes de 65 à 80 % en poids, dans chaque cas par rapport au poids total de la composition (A),
- éventuellement en outre au moins un polymère A présentant au moins dix unités constitutives de formule (I), 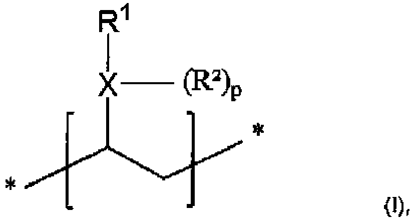 dans laquelle
- X représente l'azote ou l'oxygène, et
- R¹ et R² représentent chacun indépendamment l'hydrogène ou un groupe acyle en C2-C10 ou R¹ et R² forment, avec X, un cycle à cinq ou six chaînons, saturé ou insaturé, qui contient éventuellement d'autres hétéroatomes qui sont de préférence choisis parmi N et O, et/ou éventuellement substitués par au moins un groupe alkyle en C1-C6 et/ou par au moins un groupe fonctionnel, et
- p = 0 si X représente l'oxygène et p = 1 si X représente l'azote,
le polymère A ne contenant pas d'unités constitutives ioniques permanentes, l'au moins un polymère A étant de préférence présent en une quantité totale de 0,5 à 14 % en poids, de préférence de 1,0 à 11 % en poids, de manière particulièrement préférée de 2,0 à 10 % en poids, dans chaque cas par rapport au poids de la composition (A),
la composition (A) présentant de préférence un pH dans une plage de 3,5 à 7,1, de préférence de 4,5 à 6,5, de manière particulièrement préférée de 5,0 à 6,0, mesuré dans chaque cas à 20 °C.

13. Procédé de coloration et/ou d'éclaircissement par oxydation de fibres kératiniques, en particulier de cheveux humains, comprenant les étapes de procédé suivantes :
I. préparation d'une composition (AB) contenant
a) au moins un acide dicarboxylique saturé ayant 2 à 10 atomes de carbone et/ou au moins un sel de cet/ces acide(s),
b) au moins un acide aminé de formule (VI), 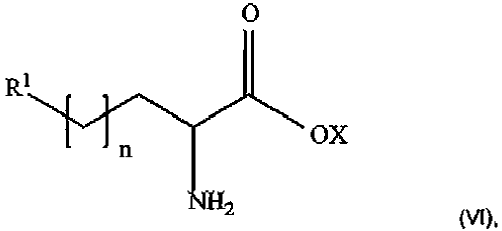 dans laquelle
X représente un atome d'hydrogène ou un cation monovalent ou divalent ;
n est égal à zéro, 1, 2 ou 3 ;
R¹ représente un radical choisi parmi un groupe amino, un groupe guanidine, un groupe (1H-imidazol-4-yl), un groupe amide d'acide carboxylique -CONH₂, un groupe 1H-indol-3-yl, un groupe thiol -SH et un groupe méthylsulfanyle -SCH₃, ou au moins un sel de cet acide aminé,
l'acide aminé de formule (VI) étant de préférence choisi parmi l'arginine, la lysine, l'histidine ainsi que des mélanges de ceux-ci, de manière particulièrement préférée les mélanges d'arginine et de lysine, ou au moins un sel de ces acides aminés,
c) éventuellement en outre au moins une substance choisie parmi
- des composés de formule générale (III), 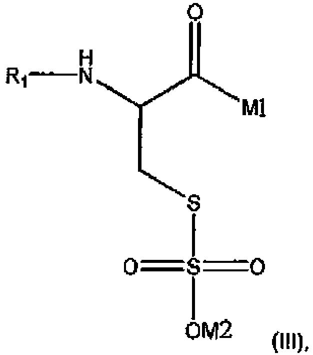 dans laquelle
R¹ représente un atome d'hydrogène ou un élément de structure de formule (IV) 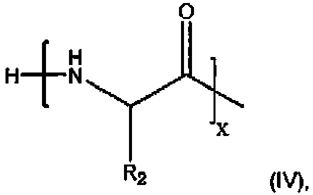 dans laquelle
x représente un nombre entier de 1 à 100,
le radical R₂ dans chacun des éléments de structure de formule (IV) peut être choisi dans chaque cas indépendamment de l'élément de structure précédent de formule (IV),
R₂ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1 H-indol-3-ylméthyle ou un groupe (sulfosulfanyl)méthyle,
M1 représente le groupement -OM2 ou un élément de structure de formule (V) 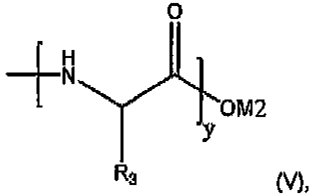 dans laquelle
y représente un nombre entier de 1 à 100,
le radical R₃ dans chacun des éléments de structure de formule (V) peut être choisi dans chaque cas indépendamment de l'élément de structure précédent de formule (V),
R₃ représente un atome d'hydrogène, un groupe méthyle, un groupe isopropyle, un groupe 2-méthylpropyle, un groupe 1-méthylpropyle, un groupe benzyle, un groupe 4-hydroxybenzyle, un groupe hydroxyméthyle, un groupe 1-hydroxyéthyle, un groupe 4-aminobutyle, un groupe 3-carbamimidamidopropyle, un groupe 2-carboxyéthyle, un groupe carboxyméthyle, un groupe 2-carbamoyléthyle, un groupe carbamoylméthyle, un groupe sulfanylméthyle, un groupe 2-(méthylsulfanyl)éthyle, un groupe 1H-imidazol-4-ylméthyle, un groupe 1H-indol-3-ylméthyle ou un groupe (sulfosulfanyl)méthyle,
M2 représente un atome d'hydrogène, un équivalent d'un cation monovalent ou polyvalent ou un ion ammonium (NH₄)⁺, et
- les polymères A qui présentent au moins dix unités constitutives de formule (1), 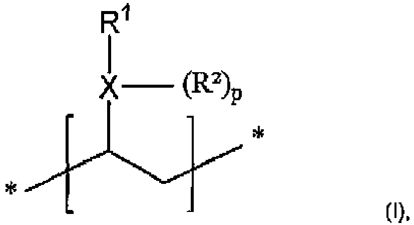 dans laquelle
- X représente l'azote ou l'oxygène, et
- R¹ et R² représentent chacun indépendamment l'hydrogène ou un groupe acyle en C2-C10 ou R¹ et R² forment, avec X, un cycle à cinq ou six chaînons, saturé ou insaturé, qui contient éventuellement d'autres hétéroatomes qui sont de préférence choisis sont parmi N et O, et/ou éventuellement substitués par au moins un groupe alkyle en C1-C6 et/ou par au moins un groupe fonctionnel, et
- p = 0 si X représente l'oxygène et p = 1 si X représente l'azote, le polymère A ne contenant pas d'unités constitutives ioniques permanentes,
d) au moins un produit d'alcalinisation choisi parmi l'hydroxyde d'ammonium, la monoéthanolamine et les silicates de sodium ainsi que des mélanges de ceux-ci,
e) de l'eau, et
f) éventuellement au moins un précurseur de colorant d'oxydation et/ou au moins un colorant direct,
II. préparation d'une composition (C) contenant
g) au moins un composé peroxyde, qui est de préférence du peroxyde d'hydrogène,
III. mélange des compositions (AB) et (C) l'une avec l'autre, immédiatement après
IV. application du mélange des compositions (AB) et (C) sur les fibres kératiniques, en particulier sur les cheveux humains, et
V. rinçage après une durée d'action de 0,1 à 60 minutes, de préférence de 1 à 45 minutes, de manière particulièrement préférée de 10 à 30 minutes,
VI. éventuellement d'autres traitements capillaires tels que le brushing, le conditionnement et/ou le séchage.

14. Procédé selon la revendication 11 ou 13, **caractérisé en ce que** l'au moins un acide dicarboxylique saturé ayant 2 à 10 atomes de carbone est choisi à partir de l'acide succinique.

15. Produit de coloration ou de décoloration selon l'une des revendications 8 à 9 ou procédé selon l'une des revendications 11, 13 ou 14 ou composition (A) selon la revendication 12, **caractérisé en ce que** l'au moins un polymère (A) présentant au moins dix unités constitutives de formule (I) et ne contenant pas d'unités constitutives ioniques permanentes, est choisi parmi la polyvinylpyrrolidone, l'alcool polyvinylique ainsi que des mélanges de ceux-ci, la polyvinylpyrrolidone étant particulièrement préférée.
